# FASCICULE DE BREVET EUROPEEN

(11) **EP 3 606 973 B1**
(45) Date de publication et mention de la délivrance du brevet: **06.09.2023**
(21) Numéro de dépôt: 18718613.5
(22) Date de dépôt: 04.04.2018
(51) Int. Cl.: C08G 18/10, C08G 18/68, C08G 18/73, C08G 18/36, A61K 8/04, A61K 8/87, A61Q 1/06, A61Q 15/00, A61Q 17/04, A61Q 19/00, C10M 119/24

(54) **NOUVEAU GELIFIANT POLYURETHANE**
NEUES POLYURETHANGELIERMITTEL
NEW POLYURETHANE GELLING AGENT

(30) Priorité: 04.04.2017 FR 1752933
(43) Date de publication de la demande: 12.02.2020
(73) Titulaire: Polymerexpert SA, 33600 Pessac (FR)
(72) Inventeur: DOLATKHANI, Marc, 33610 Cestas (FR); HECHT, Marie Odile, 33600 PESSAC (FR); LUTZ, Eric, 33600 Pessac (FR); PAGNOUX, Anne, 33114 Le Barp (FR)
(74) Mandataire: Santarelli
(86) Numéro de dépôt international: PCT/FR2018/050841
(87) Numéro de publication internationale: WO 2018/185432

(56) Documents cités:
- WO-A1-2014/072629
- WO-A1-2016/090081

## Description

L'invention concerne l'élaboration de nouveaux gels transparents en milieux organiques (huiles, solvants) à partir de nouveaux dérivés polyuréthanes, ainsi qu'un procédé pour leur préparation. L'invention concerne également les gels formés à partir de ces polyuréthanes gélifiants, ainsi que les compositions les contenant, en particulier les compositions cosmétiques.

Le procédé selon l'invention permet, en mettant en oeuvre la formation d'un dérivé isocyanate de masse molaire élevée à partir d'un estolide (composé de type polyester d'acide gras dérivés d'huiles hydroxylées), l'extension des chaines et la fonctionnalisation des bouts de chaine, d'obtenir un composé polyuréthane gélifiant apte former des gels ayant les propriétés recherchées.

L'invention a pour objet également les gels obtenus à partir de ces polyuréthanes gélifiants. Ces gels se caractérisent par leur transparence, leur faible teneur en polyuréthane gélifiant, leur capacité de dispersion à des températures inférieures à 85°C et leur caractère thermoréversible et rhéofluidifiant.

Dans le cas de la gélification d'huiles cosmétiques, le polyuréthane gélifiant doit répondre à différents critères précis. Pour des raisons de coût, lors de son utilisation dans un produit cosmétique, le polyuréthane gélifiant doit tout d'abord être capable de gélifier la phase organique à une concentration faible, c'est-à-dire inférieure à 5% massique. De plus, un aspect important pour une application cosmétique est la sensation au toucher du produit. En effet, l'incorporation du gélifiant ne doit pas induire un effet granuleux trop marqué sur le produit cosmétique.

Il est donc recherché de disposer de composés gélifiants qui possèdent ces propriétés.

L'estérification de dérivés d'huile de ricin est décrite, notamment, dans la demande WO2014/044809, pour la synthèse de pré-polymères utilisables pour la préparation de polymères utilisés comme additifs dans un matrice de poly(acide lactique). Les polyuréthanes obtenus à partir de ces estolides comprennent des groupements polyester en bout de chaine, et/ou ne comprennent pas de motif extenseur de chaine, notamment de type 1,10-décanediol. De ce fait, ils ne présentent pas les propriétés de gélification recherchées.

La demande WO2011/030075 décrit la préparation de polyuréthanes ayant une faible masse molaire à partir d'estolides d'huile de ricin. Ces composés sont utilisés pour la préparation de mousses rigides ou flexibles et ne comprennent pas de motif extenseur de chaine, et n'ont donc pas la capacité à gélifier des huiles ou des solvants.

La demande WO2016/090081 décrit la préparation de polyuréthanes utilisables pour la gélification d'huiles. Ces polyuréthanes sont préparés principalement à partir de polymères pétrosourcés (c'est-à-dire préparés à partir de produits issus de l'industrie pétrolière), comme le polybutadiène. La synthèse de polyuréthanes à partir de polyesters est envisagée, mais uniquement à partir de la réaction d'un glycol avec un ester d'acide carboxylique, et sans précision quant au caractère biosourcé (c'est à dire d'origine naturelle) ou non des monomères. De plus, cette demande décrit des réactions en une seule étape entraînant la formation possible de sous-produits insolubles dans les huiles, qui peuvent influer sur la viscosité et la transparence des gels obtenus à partir de ces polyuréthanes gélifiants en présence d'huiles.

On a maintenant trouvé que de nouveaux dérivés polyuréthanes, préparés à partir d'estolides d'origine naturelle et comprenant un motif extenseur de chaine, étaient particulièrement adaptés pour la gélification d'huiles organiques, et permettaient d'obtenir un gel ayant les qualités recherchées, notamment, pour une application cosmétique.

Par « d'origine naturelle », on entend un produit entièrement ou partiellement préparé à partir de matières d'origine naturelle.

La teneur en contenu d'origine naturelle du polyuréthane, exprimée en pourcentage pondéral, peut être calculée, notamment, selon la norme NF ISO 16128-2.

Avantageusement, ces nouveaux dérivés polyuréthanes peuvent être préparés par un procédé de synthèse comprenant une étape de fonctionnalisation d'un estolide diol par un dérivé diisocyanate, suivie d'une étape d'ajout du motif extenseur de chaine.

L'invention concerne donc des composés polyuréthanes de formule (I) 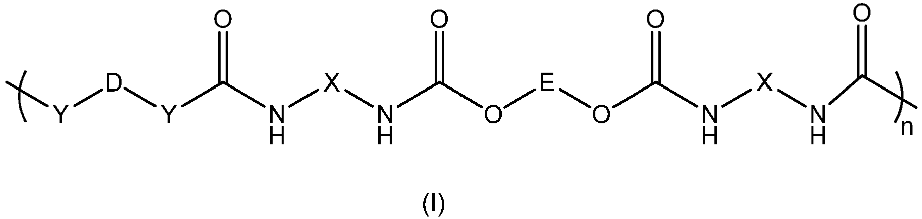 dans laquelle 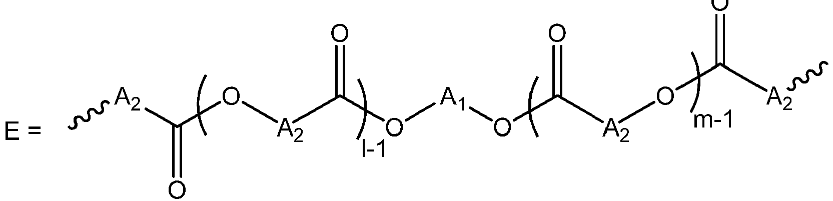
- A₁ représente un radical alkyl, linéaire ou ramifié, comprenant de 2 à 20 atomes de carbone, de préférence de 2 à 12, et en particulier de 2 à 6, ledit radical comprenant éventuellement une ou plusieurs insaturations, étant éventuellement interrompu par au moins un hétéroatome choisi parmi O, N et S, et étant éventuellement substitué par au moins un substituant -OAlk, Alk représentant un groupe alkyle comprenant de 1 à 10 atomes de carbone ;
- A₂ représente un radical alkyl, linéaire ou ramifié, comprenant de 2 à 20 atomes de carbone, de préférence de 5 à 18, et préférentiellement de 6 à 17 atomes de carbone, ledit radical comprenant éventuellement une ou plusieurs insaturations, et étant éventuellement substitué par au moins un substituant -OAlk, Alk représentant un groupe alkyle comprenant de 1 à 10 atomes de carbone ;
- l et m représentent, indépendamment l'un de l'autre, un nombre entier de 1 à 20, de préférence de 1 à 10, la somme l+m étant, de préférence, de 2 à 20 ;
- n est un nombre entier de 1 à 20, de préférence de 2 à 8 ;
- D représente un radical alkyl, linéaire ou ramifié, comprenant de 2 à 40 atomes de carbone, de préférence 6 à 14 atomes de carbone, ledit radical comprenant éventuellement une ou plusieurs insaturations;
- X est un radical alkyl linéaire ou ramifié, présentant ou non des insaturations, comprenant de 2 à 20 atomes de carbone;
- Y représente un atome ou un groupe choisi parmi: O, NH, S et C(O)O.

Parmi les composés de formule (I), ceux dans lesquels au moins une des conditions suivantes est remplie sont préférés :
- A₁ représente un radical alkyl linéaire comprenant de 2 à 12, de préférence 2 à 6, en particulier 3 atomes de carbone, ledit radical comprenant éventuellement une ou plusieurs insaturations ;
- A₂ représente un radical alkyl, linéaire ou ramifié, comprenant de 2 à 20 atomes de carbone, notamment de 12 à 18 atomes de carbone, ledit radical comprenant éventuellement une ou plusieurs insaturations, et de manière plus préférée un groupe de formule 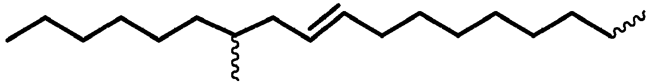
- l et m représentent, indépendamment un nombre entier de 1 à 10, la somme l+m étant, de préférence, de 2 à 20 ;
- la somme l+m a une valeur moyenne de 5 à 12, en particulier 7 ou 11,
- n est un nombre entier de 2 à 8 ;
- D est un radical alkyl, linéaire ou ramifié, comprenant de 6 à 14 atomes de carbone, ledit radical comprenant éventuellement une ou plusieurs insaturations
- X est un radical alkyl linéaire ou ramifié, présentant ou non des insaturations, comprenant de 2 à 10, en particulier 6 atomes de carbone.
- Y représente un atome d'oxygène.

Des composés polyuréthanes de formule (I) préférés sont ceux dans laquelle :
- A₁ représente un radical alkyl, linéaire ou ramifié, comprenant de 2 à 6, en particulier 3 atomes de carbone ;
- A₂ représente un radical alkyl, linéaire ou ramifié, comprenant de 12 à 18 atomes de carbone, ledit radical comprenant éventuellement une ou plusieurs insaturations ;
- l et m représentent, indépendamment l'un de l'autre, un nombre entier de 1 à 10, la somme l+m étant, de préférence, de 5 à 12 ;
- n est un nombre entier de 2 à 8 ;
- D représente un radical alkyl, linéaire ou ramifié, comprenant 6 à 14 atomes de carbone, ledit radical comprenant éventuellement une ou plusieurs insaturations ;
- X est un radical alkyl linéaire ou ramifié, présentant ou non des insaturations, comprenant de 2 à 10, en particulier 6 atomes de carbone, et
- Y représente un atome d'oxygène.

Un composé polyuréthane de formule (I) préféré est tel que défini ci-dessous :
- A₁ = C₃H₆,
- A₂ représente un groupe de formule 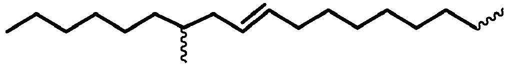
- l + m = 7 (valeur moyenne),
- n est un nombre entier de 2 à 8 ,
- D = C₁₀H₂₀,
- X = C₆H₁₂, et
- Y représente un atome d'oxygène.

L'invention concerne également un procédé de préparation de polyuréthanes gélifiants. La préparation du polyuréthane gélifiant a pour objectif de synthétiser puis de modifier chimiquement un estolide (composés de type polyester d'acide gras dérivés d'huiles hydroxylées, comme l'huile de ricin) afin d'y incorporer des groupements uréthanes permettant ainsi d'apporter les propriétés de gélification souhaitées.

Selon l'invention, ledit procédé de préparation de polyuréthanes gélifiants comprend les étapes suivantes :
1) La fonctionnalisation d'un estolide di-OH de formule (3) par un dérivé diisocyanate de formule (4) pour obtenir un estolide diisocyanate de formule (5) 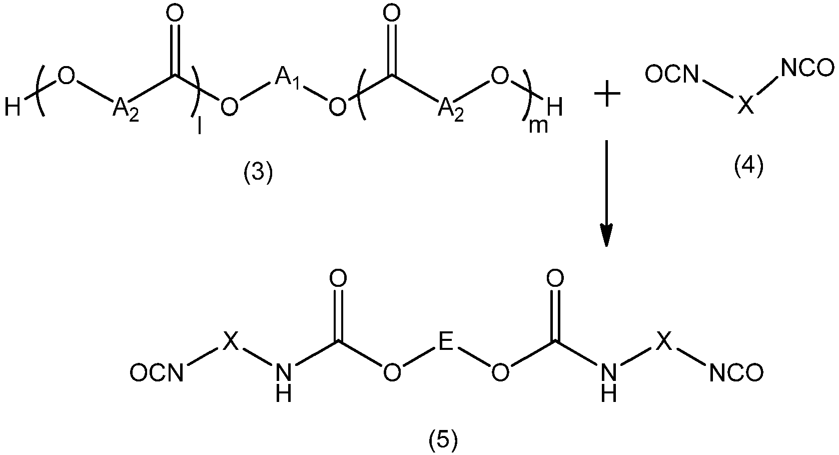 dans lesquelles : 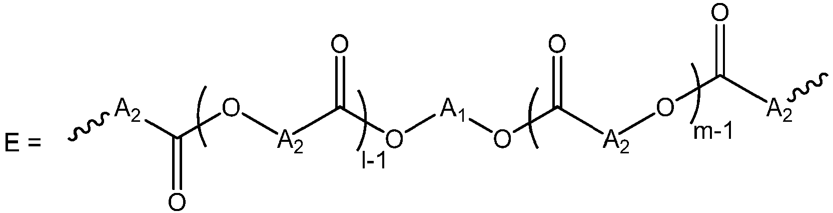
   - A₁ représente un radical alkyl, linéaire ou ramifié, comprenant de 2 à 20 atomes de carbone, de préférence de 2 à 12, et en particulier de 2 à 6, ledit radical comprenant éventuellement une ou plusieurs insaturations, étant éventuellement interrompu par au moins un hétéroatome choisi parmi O, N et S, et étant éventuellement substitué par au moins un substituant -OAlk, Alk représentant un groupe alkyle comprenant de 1 à 10 atomes de carbone ;
   - A₂ représente un radical alkyl, linéaire ou ramifié, comprenant de 2 à 20 atomes de carbone, de préférence de 5 à 18, et préférentiellement de 6 à 17 atomes de carbone, ledit radical comprenant éventuellement une ou plusieurs insaturations, et étant éventuellement substitué par au moins un substituant -OAlk, Alk représentant un groupe alkyle comprenant de 1 à 10 atomes de carbone ;
   - l et m représentent, indépendamment l'un de l'autre, un nombre entier de 1 à 20, de préférence de 1 à 10, la somme l+m étant, de préférence, de 2 à 20;
   - X est un radical alkyl linéaire ou ramifié, présentant ou non des insaturations, comprenant de 2 à 20 atomes de carbone ;
2) L'extension de chaine par ajout d'un composé difonctionnel (6) pouvant réagir avec les isocyanates du composé (5), optionnellement solubilisé dans une huile et conduisant au polyuréthane ou composé analogue (7) ayant des propriétés gélifiantes 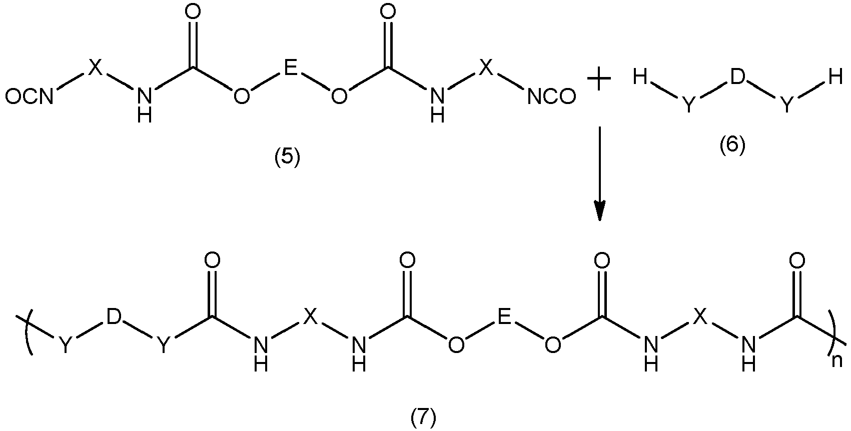 dans lesquelles
   - A₁, A₂, E, X, l et m sont tels que définis ci-dessus ;
   - D représente un radical alkyl, linéaire ou ramifié, comprenant de 2 à 40 atomes de carbone, de préférence de 6 à 14 atomes de carbone, ledit radical comprenant éventuellement une ou plusieurs insaturations;
   - n est un nombre entier de 1 à 20, notamment de 2 à 12, de préférence de 2 à 8 ;
   - Y représente un atome ou un groupe parmi: O, NH, S, C(O)O ;
   et
3) optionnellement, la terminaison de chaine par ajout d'un composé nucléophile pouvant réagir avec les fonctions isocyanates éventuellement résiduelles

De préférence, A₁ représente un radical alkyl linéaire comprenant de 2 à 12, de préférence 2 à 6, en particulier 3 atomes de carbone, ledit radical comprenant éventuellement une ou plusieurs insaturations

De préférence, A₂ représente un radical alkyl, linéaire ou ramifié, comprenant de 2 à 20 atomes de carbone, notamment de 12 à 18 atomes de carbone, ledit radical comprenant éventuellement une ou plusieurs insaturations, et de manière plus préférée un groupe de formule :

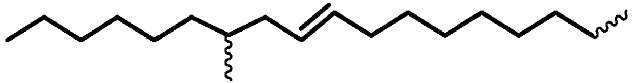

Tous les aspects préférés relatifs à A₁, A₂, E, X, l, m, n, D, X et Y mentionnés plus haut pour les composés polyuréthanes de formule (I) s'appliquent également au procédé de préparation selon l'invention.

Le diisocyanate de formule (4) peut être choisi, par exemple, parmi les diisocyanates aliphatiques non ramifiés comprenant 2 à environ 36 atomes de carbone, ou d' de 4 à 30 atomes de carbone, tels que, par exemple, l'hexaméthylène diisocyanate (HMDI), le 1,10-décane diisocyanate, le 1,4 butane diisocyanate (BDI) ou le 1,12-dodécane diisocyanate.

Un diisocyanate préféré aux fins de l'invention est l'hexaméthylène diisocyanate (HMDI).

### L'estolide diol de formule (3)

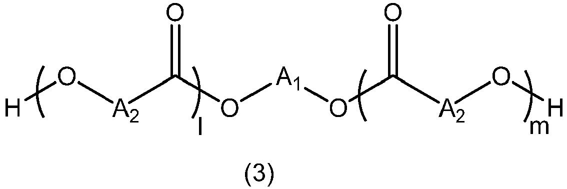

dans laquelle A₁, A₂, l et m sont tels que définis ci-dessus, est disponible commercialement, ou peut être préparé par condensation d'un diol (1) avec un dérivé d'acide gras hydroxylé (2) : 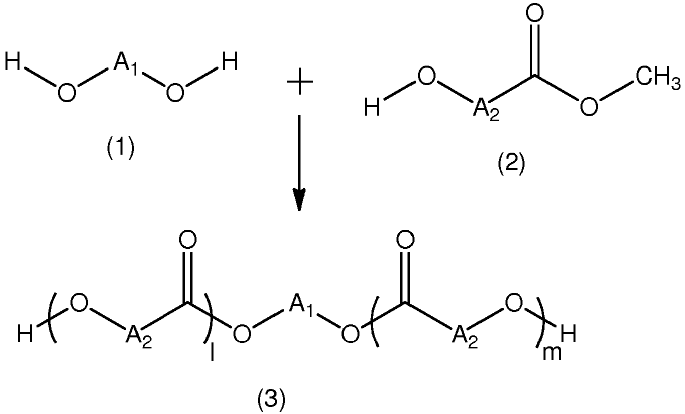
comme décrit, par exemple, dans la demande WO2014/044809.

On peut se référer par exemple, plus particulièrement, à la préparation des composés de formule (I-2) et (IA-2). La synthèse est décrite notamment dans l'étape 1 de l'exemple 1.

La 1^{ere} étape, à savoir l'étape de fonctionnalisation de l'estolide di-OH par un dérivé diisocyanate a, de préférence, une durée comprise entre 15 min et 6h, de préférence entre 30 min et 2h30, et est effectuée en présence ou non d'un solvant de type huile.

Selon le composé final souhaité, cette étape de fonctionnalisation de l'estolide di-OH par un dérivé diisocyanate peut être effectuée, par exemple, selon 2 alternatives, en présence ou non d'un solvant de type huile, tel que, par exemple, le triglycéride caprylique/caprique, à savoir :
- par ajout rapide (durée inférieure à environ 1 min) du diisocyanate sur l'estolide diOH (Etape 1a), ou
- par ajout lent (durée d'environ 15 min) ou rapide (durée inférieure à environ 1 min) de l'estolide diOH sur le diisocyanate, (1b).

L'étape 1a favorise la formation d'oligomères d'estolide, alors que l'étape 1b favorise la fonctionnalisation des bouts de chaine des estolides.

L'étape 1a peut être réalisée en ajoutant rapidement une quantité de diisocyanate (4) comprise entre 1,4 et 2 équivalents dans un réacteur avec agitation mécanique contenant 1 équivalent (eq) d'estolide di-OH (3), calculé d'après son indice d'OH
La réaction est réalisée à une température comprise entre 60°C et 150°C, de préférence entre 110°C et 135°C, notamment entre 115°C et 135°C, dans un système étanche préalablement séché et placé sous un flux continu d'azote. L'étape 1b peut être réalisée en ajoutant 1 équivalent (eq) d'estolide di-OH (3) en l'espace de 15 min sur un mélange d'huile (par exemple, du triglycéride caprylique/caprique) et de diisocyanate (4) présent en quantité comprise entre 1,4 à 2,0 équivalents dans un réacteur avec agitation mécanique. La réaction est réalisée à une température supérieure ou égale à 110°C dans un réacteur étanche préalablement séché et placé sous un flux continu d'azote.

La 2^{ème} étape du procédé selon l'invention (synthèse du polyuréthane d'estolide de diol) peut être effectuée à une température supérieure à 110°C, notamment supérieure à 115°C de préférence comprise entre 110°C et 130°C, notamment entre 115°C et 130°C, pendant une durée comprise entre 5 minutes et 2 heures, par ajout d'un ou plusieurs molécules difonctionnelles nucléophiles pouvant être ou non solubilisé dans un solvant de type huile. Le ou les nucléophile(s), est (sont) ajoutés à l'estolide diisocyanate afin de former le gélifiant de type polyuréthane. Le ratio entre le nucléophile difonctionnel (6) et l'estolide diisocyanate (5) est un des facteurs qui influe sur les capacités de gélification du produit final. On utilisera, de préférence une quantité de diol compris entre 0,1 et 2 équivalent(s), et plus particulièrement entre 0,4 et 0,8 équivalent (eq) pour 1 eq d'estolide diisocyanate. Un diol préféré est 1,10-décanediol. D'autres huiles cosmétiques ou non ayant comme propriété de solubiliser le 1,10-décanediol à chaud peuvent être utilisés lors de cette étape.

A la fin de la 2^{ème} étape, le polyuréthane gélifiant est déjà formé mais peut éventuellement présenter encore des fonctions isocyanates résiduelles. Pour éliminer ces fonctions isocyanates n'ayant pas réagi et terminer la réaction, une molécule nucléophile de type alcool, thiol, amine ou carboxylate, monofonctionnelle ou difonctionnelle, est ajoutée en excès au milieu réactionnel au cours d'une 3^{ème} étape (optionnelle) à une température comprise entre 110 et 150°C, notamment entre 115°C et 130°C. Cette molécule nucléophile est idéalement soluble dans les huiles cosmétiques.

A l'issue de la 3^{ème} étape, la récupération du polyuréthane gélifiant peut être effectuée, par exemple, en le faisant couler à une température supérieure à 110°C du réacteur vers un récipient adapté.

Le procédé selon l'invention présente plusieurs nouveautés et avantages par rapport à ceux de l'art antérieur. En particulier, ce procédé se caractérise par des temps de réaction courts, ainsi que par l'absence de formation de produits secondaires difficilement solubles dans les huiles, renforçant ainsi la transparence des gels une fois le polyuréthane gélifiant dissous dans une huile. De plus, il fait intervenir majoritairement des composés d'origine naturelle.

L'invention concerne également l'utilisation du polyuréthane gélifiant selon l'invention, ou obtenu selon le procédé de l'invention, comme agent gélifiant de milieux organiques. En particulier, l'invention concerne également l'utilisation du polyuréthane gélifiant selon l'invention, ou obtenu selon le procédé de l'invention, comme agent gélifiant d'huiles choisies, par exemple, parmi les huiles organiques, dont les huiles végétales, et les mélanges d'huiles apolaires et d'huiles plus polaires, telles que, par exemple hydrogenated polyisobutene, polyisobutene, etc. (dénomination INCI).

L'invention concerne également un procédé de gélification d'au moins une huile choisie, par exemple, parmi les huiles organiques et les mélanges d'huiles apolaires et d'huiles plus polaires, dans lequel un polyuréthane gélifiant de formule (I) tel que décrit plus haut, selon l'invention, ou obtenu par le procédé de préparation décrit plus haut, est ajouté à ladite ou auxdites huile(s), ou à une composition la ou les contenant.

Les gels comprenant le polyuréthane gélifiant selon l'invention, ou obtenu selon le procédé de l'invention, et au moins une huile acceptable sur le plan pharmaceutique ou cosmétique sont un objet de l'invention.

Selon un aspect préféré, le polyuréthane gélifiant selon l'invention, ou obtenu selon le procédé de l'invention permet de former, par exemple, un gel fort très rhéofluidifiant et thixotrope à une teneur de 5% massique dans du triglycéride caprylique/caprique. Lorsqu'on applique un taux de cisaillement constant de 100s⁻¹ pendant 5 minutes à ce gel, la viscosité diminue de 2,2 à 1,2 Pa.s. Ce gel reprend ensuite sa viscosité initiale en 8 heures.

Les polyuréthanes gélifiants selon l'invention permettent de former des gels dans de nombreuses huiles cosmétiques, telles que, par exemple : C12-C15 alkylbenzoate, dimer dilinoleyl dimer dilinoleate, caprylic/capric triglycéride, isononyl isononanoate, Ricinus communis seed oil, triethylhexanoin, ethylhexyl palmitate, ethylhexyl myristate, dicaprylyl carbonate, isopropyl myristate, isopropyl palmitate, propylene glycol, dicaprylyl carbonate, pentaerythrityl tetraisostearate, ethylhexyl stéarate, bis diglyceryl polyacyladipate, Triticum vulgare germ oil, Prunus amygdalus dulcis, Glycine soja oil, Sunflower helianthus oil, Sesamum indicum oil, Butyrospermum Parkii (Shea) Butter, Coco Nucifera (Coconut) Oil, etc. (dénominations INCI). Les polyuréthanes gélifiants selon l'invention peuvent également gélifier les huiles apolaires si elles sont mélangées à des huiles plus polaires, comme, par exemple, un mélange Hydrogenated Polyisobutene 50%/isopropyle myristate 50%.

Le polyuréthane gélifiant selon l'invention, ou obtenu selon le procédé de l'invention, comme agent gélifiant peut également être utilisé comme agent gélifiant de glycols, tels que la glycérine, le propylène glycol ou le butylène glycol, notamment PEG-40 sorbitan peroleate, PEG-20 Glyceryl Triisostearate etc.

Egalement, le polyuréthane gélifiant selon l'invention, ou obtenu selon le procédé de l'invention permet de gélifier des huiles et de stabiliser des mélanges contenant jusqu'à 8% de cires avec les cires suivantes : cire d'abeille, C10-18 triglycérides, behenyl alcohol, glyceryl stéarate, synthetic wax, pentaerythrityl distearate, copernica cerifera wax, cetearyl alcohol, cetyl alcohol, ozokerite, rice bran wax, sunflower wax, bis diglyceryl polyacyladipate (dénomination INCI).

Le polyuréthane gélifiant permet également de gélifier des huiles et de stabiliser des mélanges contenant jusqu'à 8% en poids de beurres avec, par exemple, les beurres suivants : beurre de karité, beurre d'argan, beurre d'abricot, beurre de coco.

L'invention concerne également les gels contenant ou formés à partir du polyuréthane gélifiant selon l'invention, ou obtenu selon le procédé de l'invention.

La teneur massique du polyuréthane gélifiant dans ledit gel peut être, par exemple, comprise entre 0,7% et 10%, de préférence de 1 à 5%.

L'invention a également pour objet les compositions comprenant les gels contenant ou formés à partir du polyuréthane gélifiant tel que décrit ci-dessus, en particulier les compositions pharmaceutiques ou cosmétiques.

Lesdites compositions pharmaceutiques ou cosmétiques peuvent comprendre au moins un ingrédient actif et au moins un véhicule et/ou additifs pharmaceutiquement acceptables ou acceptables en cosmétique.

La teneur massique du polyuréthane gélifiant dans une composition cosmétique selon l'invention peut être, par exemple, comprise entre 0,7% et 10%, de préférence de 1 à 5%.

Les polyuréthanes gélifiants selon l'invention, ou obtenus selon le procédé de l'invention, peuvent donc être associés en cosmétique avec des huiles polaires, apolaires, végétales, des glycols, des parfums.

Ils sont, en particulier, compatibles avec les filtres solaires organiques et minéraux, les pigments et les nacres, les actifs huileux couramment utilisés en cosmétique, les beurres et les cires cosmétiques ou les émulsionnants.

Les polyuréthanes gélifiants décrits ci-dessus peuvent être incorporés dans des formulations galéniques variées anhydres telles que gel anhydre, gel vaporisable, gel nacré, gel teinté, gel solaire organique, gel solaire inorganique, gel parfumé, baume à lèvres, gloss, gel démaquillant huileux, stick, etc.

Ils peuvent également être incorporés dans des formulations de type émulsion ; telle que, par exemple, un gel crème sans émulsionnant, une émulsion Eau dans huile, une émulsion Huile dans eau, un mascara, un fond de teint, une mousse, etc...

L'invention est illustrée par les exemples suivants.

Dans les exemples, les estolides de propane-1,3-diol (PDO) PRIC212 et PRIC2205H sont des polyricinoléates de méthyle commercialisés par l'ITERG, (Institut des Corps Gras). Le HMDI est de l'hexaméthylène diisiocyanate (Covestro). Le Labrafac CC (Nom INCI : Caprylic/Capric Triglycéride) provient de chez Gattefossé. Le 1,10-décanediol provient de Beckmann-Kenko GmbH.

L'estolide de 1,3-propanediol diOH (3) peut également être préparé comme décrit dans la « Préparation 1 » ci-dessous.

### Préparation 1 : Synthèse de l'estolide de 1,3-propanediol diOH (3)

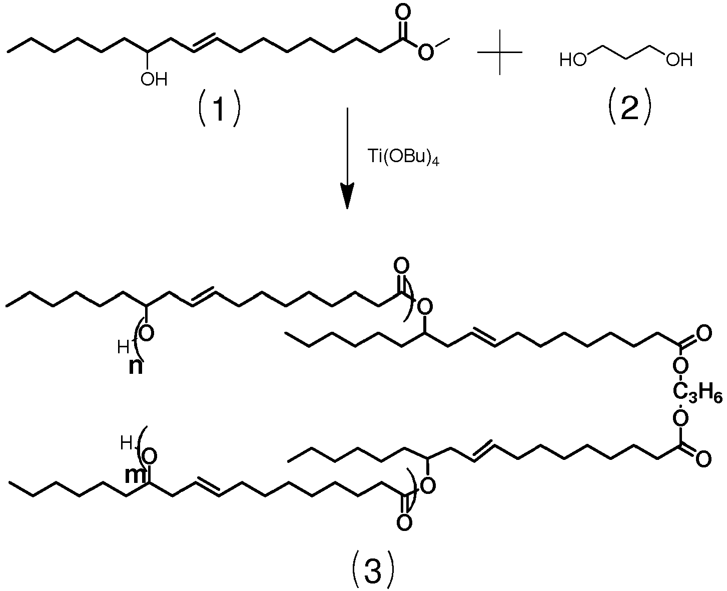

Le poly(ricinoléate de méthyle) dihydroxytéléchélique (3) a été synthétisé par transestérification du ricinoléate de méthyle (1) en présence de 1,3-propanediol ainsi que de Ti(OBu) comme décrit dans la demande WO2014/044809. Le ricinoléate de méthyle (1) utilisé a été préalablement purifié sur colonne chromatographique en utilisant comme éluant un mélange Heptane/Acétone (v/v : 98/2). Le produit (1), après purification, possède une pureté de 99% après analyse par chromatographie en phase gazeuse.

Dans un ballon de 50 mL, 5 g de ricinoléate de méthyle (1), 73 mg de 1,3-propanediol (2) ainsi que 54 mg de Ti(OBu) ont été introduits. Le mélange réactionnel a été laissé sous agitation 140°C pendant 2 h sous flux de N₂. La température a ensuite été élevée à 180°C pendant 1 h puis le ballon a été placé sous vide dynamique pendant 21 h à 180°C. A la fin de la réaction, le polymère a été dissous dans du dichlorométhane puis précipité dans du méthanol et enfin séché sous pression réduite jusqu'à stabilisation de la masse.

### Exemple 1 : Préparation d'un polyuréthane d'estolide de PDO (PRIC212) et de 1,10-décanediol dans laquelle les étapes 1) et 2) sont réalisées à deux températures différentes (GSC16179)

Le polyuréthane gélifiant a été synthétisé en 3 étapes par polyaddition de l'estolide de PDO diisocyanate avec du 1,10-décanediol en présence d'une huile cosmétique. Il présente un contenu d'origine naturel de 92,8% selon la norme NF ISO 16128-2.

### 1) Synthèse de l'estolide de 1,3-propanediol diisocyanate (5)

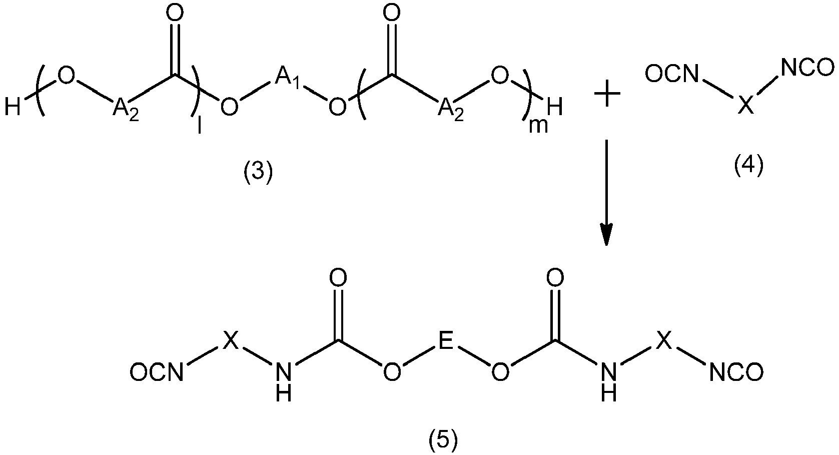

avec : 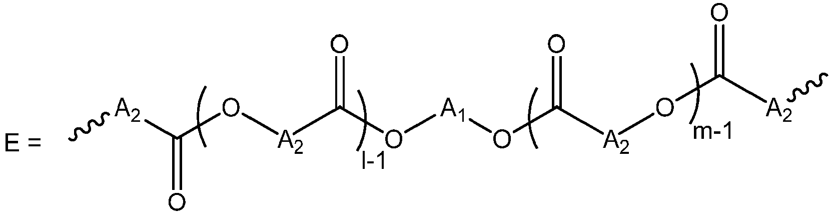
- X = C₆H₁₂
- A₁ = C₃H₆
- A₂ représente un groupe de formule 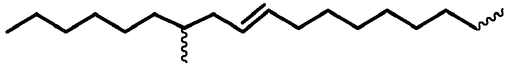 - l + m = 7 (valeur moyenne)

Dans un réacteur de 1 L, 379 g d'estolide de 1,3-propanediol PRIC212 (3) obtenu à l'étape 1 séché sous vide pendant 2h15 à 90°C et 58 g de HMDI (4) ont été introduits par ajout rapide (< 1 min) de (4) sur (3). Le mélange réactionnel a été laissé sous agitation mécanique à 60°C pendant 2,5 h sous flux d'azote.

### 2) Synthèse du polyuréthane d'estolide de 1,3-propanediol (7)

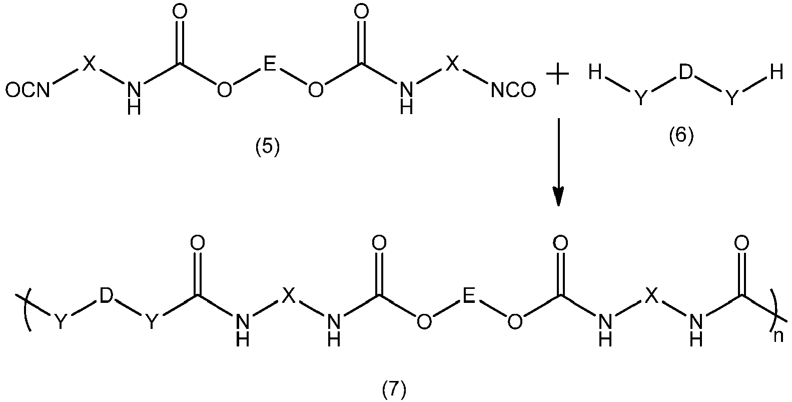

Avec :
- D = C₁₀H₂₀
- Y = 0

21,2 g de 1,10-décanediol et 162 g de Labrafac CC (triglycéride caprylique/caprique) sont ajoutés au milieu réactionnel, puis la température est augmentée à 130°C. La réaction est poursuivie pendant 30 min après la dissolution du 1,10-décanediol sous flux d'azote.

### 3) Synthèse du polyuréthane gélifiant (8)

Pour éliminer les fonctions isocyanate n'ayant pas réagi et terminer la réaction, 188 g d'estolide de 1,3-propanediol diOH (3) est ajouté à 130°C dans le réacteur de 1L de l'étape 3 contenant le polyuréthane d'estolide de 1,3-propanediol (7). La réaction est prolongée pendant 1 h jusqu'à disparition complète du pic lié au NCO en IR (pic à 2250cm-1).

### Exemple 2 : Préparation d'un polyuréthane d'estolide de PDO (PRIC212) et de 1,10-décanediol dans laquelle les étapes 1) et 2) sont réalisées à la même température (GSC17016)

Le polyuréthane gélifiant a été synthétisé en 3 étapes par polyaddition de l'estolide de PDO diisocyanate avec un extenseur de chaine en présence d'une huile cosmétique. Il présente un contenu d'origine naturelle de 92,2% selon la norme NF ISO 16128-2.

### 1) Synthèse de l'estolide de 1,3-propanediol diisocyanate (5)

Dans un réacteur de 1L, 103 g de Labrafac CC (triglycéride caprylique/caprique) est chauffé à 80°C sous flux d'azote et sous agitation mécanique (500 rpm). Du HMDI (40 g) est ensuite ajoute au mélange et la température est augmentée à 115°C. 20 min après l'ajout du HMDI, de l'estolide de propanediol PRIC212 (Mn 2000 g/mol, 238 g) est ajouté en 15 min par ampoule de coulée en maintenant la température à 115°C sous flux d'azote. La réaction est poursuivie pendant 1h15.

### 2) Synthèse du polyuréthane d'estolide de 1,3-propanediol (7)

15,5 g de 1,10-décanediol sont ajoutés au milieu réactionnel à 115 °C, et la réaction est poursuivie pendant 45 min après l'ajout.

### 3) Terminaison de la réaction

Pour éliminer les fonctions isocyanate n'ayant pas réagi et terminer la réaction, 119 g d'estolide de 1,3-propanediol diOH PRIC212 (Mn 2000g/mol) sont ajoutés dans le réacteur de 1 L. La réaction est prolongée pendant 1 h jusqu'à disparition complète en IR du pic lié aux fonctions NCO.

### Exemple 3 : Des essais de gélification de différentes huiles ont été effectués avec les polyuréthanes gélifiants des exemples 1 et 2 (5% en masse).

Les résultats ont rapportés dans le tableau 1 ci-dessous. Les huiles sont identifiées par leur dénomination INCI.

Le gélifiant a été dispersé dans l'huile à 130°C sous agitation.

La force des gels a été évaluée de la manière suivante :
La transparence des gels est évaluée qualitativement, par observation d'un pilulier contenant du gel. S'il est possible de lire des caractères écrits de façon très nette à travers le pilulier, alors le gélifiant est considéré comme formant des gels transparents. Le gel est considéré comme fort s'il ne s'écoule pas lorsque le pilulier est renversé.

**Tableau 1**

| **Huiles (INCI)** | **Formation de gels (Exemple 1)** | **Formation de gels (Exemple 2)** |
|---|---|---|
| Isopropyl myristate | Gel fort transparent | Gel fort transparent |
| Dicaprylyl carbonate | Gel fort transparent | Gel fort transparent |
| Pentaerythrityl tetraisostearate | Gel fort transparent | Gel fort transparent |
| C12-C15 alkyl benzoate | Gel fort transparent | Gel fort transparent |
| Caprylic/capric triglycéride | Gel fort transparent | Gel fort transparent |
| Ethylhexyl palmitate | Gel fort transparent | Gel fort transparent |
| Isononyl isononanoate | Gel fort transparent | Gel fort transparent |
| Isopropyl palmitate | Gel fort transparent | Gel fort transparent |
| Ricinus communis seed oil | Gel fort transparent | Gel fort transparent |
| Dimer dilinoleyl dimer dilinoleate | Gel fort transparent | Gel fort transparent |

### Exemple 4 : Mesure de la viscosité et de la température de liquéfaction de gels réalisés dans le Caprylic/Capric triglycéride avec les polyuréthanes des exemples 1 et 2.

Les mesures de viscosité ont été réalisées avec un viscosimètre TA Instruments AR1500ex par mesure de rhéologie en écoulement, à des taux de cisaillement de 100 s⁻¹.

La température de liquéfaction a été mesurée en oscillation à 1 Pa et 1 Hz.

Les résultats montrent une viscosité importante des gels dès la présence de 2% de gélifiant dans l'huile, et une température de liquéfaction supérieure à 50°C, permettant leur utilisation en cosmétique.

**Tableau 2**

| **Exemple** | **Numéro de lot** | **Viscosité en Pa.s** (cisaillement 100^{s-1} à 20 °C, mesure à t=10s) | | **Température de liquéfaction (°C)** (oscillation, 1 Pa, 1 Hz) | |
|---|---|---|---|---|---|
| | | Gel 5% dans caprylic/capric triglycéride | Gel 2% dans caprylic/capric triglycéride | Gel 5% dans caprylic/capric triglycéride | Gel 2% dans caprylic/capric triglycéride |
| 1 | GSC16179 | 1,33 | 0,38 | Non testé | 68,5 |
| 2 | GSC17016 | 1,01 | 0,26 | 76,3 | 65,1 |

### Exemple 5 : Synthèse d'un polyuréthane d'estolide de 1,3-propanediol, de 1,10-décanediol et d'huile de ricin (GSC16162)

Le polyuréthane gélifiant a été synthétisé en 3 étapes par polyaddition de l'estolide de 1,3-propanediol diisocyanate avec du 1,10-décanediol en présence de deux huiles cosmétiques. Il présente un contenu d'origine naturelle de 95,0% selon la norme NF ISO 16128-2.

### 1) Synthèse de l'estolide de 1,3-propanediol diisocyanate (5)

Dans un réacteur de 1 L, 194,7 g d'estolide de 1,3-propanediol (3) et 39,5 g de HMDI (4) ont été introduits. Le mélange réactionnel a été laissé sous agitation mécanique à 60°C pendant 2,5 h sous flux d'azote. Des mesures infrarouges ont été effectuées en contrôlant le ratio de fonction -NCO (pic à 2250 cm-1) par rapport aux fonctions -CH (plusieurs bandes entre 2800 et 3000 cm-1). La réaction est poursuivie jusqu'à ce que le ratio NCO/CH soit constant sur au moins deux mesures séparées de 15 min.

### 2) Synthèse du polyuréthane d'estolide de 1,3-propanediol (7)

14,5 g de 1,10-décanediol sont solubilisés à 130°C dans 86,1 g de Labrafac CC (triglycéride caprylique/caprique, Gattefossé). La température du réacteur de 1L de l'étape 1 contenant l'estolide de 1,3-propanediol diisocyanate (5) est augmentée à 130°C, puis le mélange 1,10-décanediol (6) plus Labrafac CC est ajouté en une fois dans le milieu réactionnel. La réaction est poursuivie pendant 30 min sous flux d'azote.

### 3) Synthèse du polyuréthane gélifiant (8)

Pour éliminer les fonctions isocyanates n'ayant pas réagi et terminer la réaction, 463 g d'huile de ricin est ajouté en excès à 130°C dans le réacteur de 1L de l'étape 3 contenant le polyuréthane d'estolide de 1,3-propanediol (7). La réaction est prolongée 1 h jusqu'à disparition complète du pic lié au NCO en IR.

### Exemple 6 : Synthèse d'un polyuréthane d'estolide de PDO et d'hexanediol (GSC16080)

Le polyuréthane gélifiant a été synthétisé en 3 étapes par polyaddition de l'estolide de PDO diisocyanate avec du 1,6-hexanediol en présence d'une huile cosmétique. Il présente un contenu d'origine naturelle de 92,7% selon la norme NF ISO 16128-2.

### 1) Synthèse de l'estolide de 1,3-propanediol diisocyanate (5)

Dans un ballon de 100 mL, 33,2 g d'estolide de 1,3-propanediol PRIC212 (Mn 2000 g/mol) (3) et 7,2 g de HMDI (4) ont été introduits. Le mélange réactionnel a été laissé sous agitation mécanique à 60°C pendant 3,2 h sous flux d'azote.

### 2) Synthèse du polyuréthane d'estolide de 1,3-propanediol (7)

9,72 g d'estolide de 1,3-propanediol diisocyanate sont prélevés et placés dans un ballon de 100 mL. 0,48 g de 1,6-hexanediol sont solubilisés à 130°C dans 9,8 g de Labrafac CC (triglycéride caprylique/caprique, Gattefossé). La température du ballon de 100 mL contenant l'estolide de 1,3-propanediol diisocyanate (5) est augmentée à 130°C, puis le mélange 1,6-hexanediol (6) plus Labrafac CC est ajouté en une fois dans le milieu réactionnel. La réaction est poursuivie pendant 2h40 sous flux d'azote.

### 3) Synthèse du polyuréthane gélifiant (8)

Pour éliminer les fonctions isocyanates n'ayant pas réagi et terminer la réaction, 3,28 g d'estolide de 1,3-propanediol est ajouté en excès à 130°C dans le ballon de 100L de l'étape 2 contenant le polyuréthane d'estolide de 1,3-propanediol (7). La réaction est prolongée 2,5 h jusqu'à disparition complète du pic lié au NCO en IR.

### Exemple 7 : Synthèse d'un polyuréthane d'estolide de PDO et de 1,12-dodecanediol (GSC16071C)

Le polyuréthane gélifiant a été synthétisé en 3 étapes par polyaddition de l'estolide de PDO diisocyanate avec du 1,12-dodécanediol en présence d'huile cosmétique. Il présente un contenu d'origine naturelle de 93,4% selon la norme NF ISO 16128-2.

### 1) Synthèse de l'estolide de 1,3-propanediol diisocyanate (5)

Dans un réacteur de 250 mL, 35,7 g d'estolide de 1,3-propanediol PRIC212 (Mn 2000g/mol) (3) et 8,0g de HMDI (4) ont été introduits. Le mélange réactionnel a été laissé sous agitation mécanique à 60°C pendant 1 h sous flux d'azote puis le mélange est tiré sous vide pendant 4h30. On ajoute 43,7 g deLabrafac CC (caprylic/capric triglycéride).

### 2) Synthèse du polyuréthane d'estolide de 1,3-propanediol (7)

21,2 g du mélange précédemment obtenu d'estolide de 1,3-propanediol diisocyanate (5) et de Labrafac CC sont prélevés puis ajoutés dans un ballon de 250 mL à 130°C, puis 0,78 g de 1,12-dodécanediol sont ajoutés. La réaction est poursuivie pendant 1h sous flux d'azote.

### 3) Synthèse du polyuréthane gélifiant (8)

Pour éliminer les fonctions isocyanates n'ayant pas réagi et terminer la réaction, 10 mL d'éthanol sont ajoutés en excès à 130°C dans le ballon de 250 mL contenant le polyuréthane d'estolide de 1,3-propanediol (7). La réaction est prolongée 1 h jusqu'à disparition complète du pic lié au NCO en IR. L'éthanol est ensuite évaporé sous vide pendant 1 h.

### Exemple 8 : Les essais de gélification d'huiles avec les polyuréthanes des exemples 5, 6 et 7 (5% en masse de polyuréthane gélifiant) sont rapportés dans le tableau 3 ci-dessous.

**Tableau 3**

| **Huiles (INCl)** | **Exemple 3 - 4 - 5** |
|---|---|
| Isopropyl myristate | Gel fort transparent |
| Caprylic/capric triglycéride | Gel fort transparent |

### Exemple 9 : Synthèse d'un polyuréthane d'estolide (PRIC2205H) et de 1,10-décanediol par ajout de l'estolide sur le diisocyanate (LZE17009)

Ce polyuréthane présente un contenu d'origine naturelle de 95,7% selon la norme NF ISO 16128-2.

### 1) Synthèse de l'estolide PRIC2205H diisocyanate

Dans un ballon de 250 mL, 10 g d'estolide PRIC2205H (Mn 3000 g/mol) est ajouté à la goutte à goutte en 15 min dans un mélange contenant 0,86 g de HMDI et 4 g de Labrafac CC (triglycéride caprylique / caprique). Le mélange réactionnel a été laissé sous agitation magnétique à 115°C pendant 1,5 h sous flux d'azote.

### 2) Synthèse du polyuréthane de PRIC2205H et de1,10-décanediol

0,29 g de 1,10-décanediol sont ajoutés dans le mélange réactionnel, en maintenant la température à 115°C. La réaction est poursuivie pendant 45 min.

### 3) Synthèse du polyuréthane gélifiant

Pour éliminer les fonctions isocyanate n'ayant pas réagi et terminer la réaction, 5 g d'estolide PRIC2205H sont ensuite ajoutés en excès dans le réacteur de 250mL. La réaction est prolongée 1 h jusqu'à disparition complète du pic lié au NCO en IR.

### Exemple 10 : Synthèse d'un polyuréthane d'estolides PRIC2205H et PRIC212, et de 1,10-décanediol par ajout des estolides sur le diisocyanate (LZE17012)

Ce polyuréthane présente un contenu d'origine naturelle de 94,6% selon la norme NF ISO 16128-2.

### 1) Synthèse des estolides PRIC2205H et PRIC212 diisocyanate

Dans un ballon de 250 mL, 1,3 g d'estolide PRIC212 (Mn 2000 g/mol) puis 10 g d'estolide PRIC2205H (3000 g/mol) sont ajoutés au goutte à goutte en 15 min dans un mélange contenant 1,1 g de HMDI et 4 g de Labrafac CC (triglycéride caprylique / caprique). Le mélange réactionnel a été laissé sous agitation magnétique à 115°C pendant 1,5 h sous flux d'azote.

### 2) Synthèse du polyuréthane de PRIC2205H, de PRIC212 et de 1,10-décanediol

0,42 g de 1,10-décanediol sont ajoutés dans le mélange réactionnel, en maintenant la température à 115°C. La réaction est poursuivie pendant 45 min.

### 3) Synthèse du polyuréthane gélifiant

Pour éliminer les fonctions isocyanate n'ayant pas réagis et terminer la réaction, 3,25 g d'estolide PRIC212 sont ensuite ajoutés dans le réacteur de 250 mL. La réaction est prolongée 1 h jusqu'à disparition complète du pic lié au NCO en IR.
Les essais de gélification d'huiles avec les polyuréthanes des exemples 9 et 10 (5% en masse de polyuréthane gélifiant) sont rapportés dans le tableau 4 ci-dessous.

**Tableau 4**

| **Huiles (INCl)** | **Exemple 9** | **Exemple 10** |
|---|---|---|
| Caprylic/capric triglycéride | Gel faible | Gel faible |
| PPG 15 Stearyl Ether | Gel fort | Gel fort |
| Dicaprylyl Carbonate | Gel faible | Gel faible |
| Squalane | Gel faible | Gel fort |

### Exemple 11 : Influence des conditions de synthèse des polyuréthanes sur la viscosité de gels obtenus par dissolution des polyuréthanes dans du triglycéride caprylique / caprique.

Les mesures de viscosités effectuées en écoulement à un taux de cisaillement de 100 s⁻¹ et obtenues au bout de 10 s, sont rapportées dans le tableau 5 ci-dessous.

**Tableau 5**

| Synthèse | GSC16179 (Exemple 1) | GSC17012 (Exemple 2) |
|---|---|---|
| Viscosité (Pa.s) - **5%PU** | 1,33 | 1.08 |
| T° liq (°C) **- 5%PU** | 78,3 | 78,4 |
| Viscosité (Pa.s) - **2%PU** | 0,38 | 0.22 |
| T° liq (°C) - 2%**PU** | 69 | 67 |
| Viscosité (Pa.s) - **1%PU** | 0,10 | Non mesuré |

Il est possible de changer le ratio entre le PRIC212 (ici entre l'exemple 1 et l'exemple 2) et le HMDI pour faire varier les propriétés des gels qui seront formés avec les gélifiants.

Il est également possible de modifier les quantités de décanediol lors de l'étape 2 pour modifier de façon importante les propriétés de gélification des polyuréthanes, faisant par exemple augmenter de 1 à 1,6 Pa.s la viscosité de gels à 5% de polyuréthane dans le Caprylic/Capric Triglycéride.

### Exemple 12 : Incorporation des polyuréthanes gélifiants dans des formulations cosmétiques

Différentes formules cosmétiques sont données ci-dessous.

Pour toutes les formules cosmétiques, le gel a été préparé de la manière suivante : le polyuréthane gélifiant ajouté à l'huile est d'abord mis à température dans un bain puis soumis à l'agitation forte de l'Ultra-Turrax^{®} et enfin à une agitation par une pâle défloculeuse tout en maintenant la température à 80°C.

Les ingrédients sont nommés par leur dénomination INCI.

### 1) Formule de parfum

| INCI | % |
|---|---|
| Caprylic/capric triglyceride | 43,525 |
| **PU GSC16179 (Exemple 1)** | **12,50** |
| Parfum | 43,525 |
| Diethylhexyl Syringylidenemalonate (and) Caprylic/Capric Triglyceride | 0,05 |
| Octocrylene | 0,20 |
| Butyl Methoxydibenzoylmethane | 0,20 |

Le polyuréthane gélifiant est compatible avec les parfums couramment utilisés en cosmétique et en parfumerie.

Le polyuréthane gélifiant (par exemple lot GSC16179 [Exemple 1]) gélifie des parfums et forme des gels solides avec des teneurs en parfum pur comprises entre 0,2 et 43,5%. Ces essais ont été réalisés avec 10 parfums différents.

Le polyuréthane gélifiant présente également un pouvoir suspensif pour maintenir, par exemple, des nacres, des agents exfoliants, des poudres de densité variables dans le milieu.

### 2) Formule d'huile gélifiée avec nacres en suspension :

| INCI | % |
|---|---|
| Caprylic/capric triglyceride | 97,8 |
| **PU GSC16179 [Exemple 1]** | **1** |
| Synthetic Fluorphlogopite (and) Iron Oxide | 0,1 |
| Parfum | 0,1 |
| Caryodendron orinocense Nut Oil | 1 |

Le polyuréthane gélifiant forme des gels transparents permettant de mettre des éléments en suspension dès 1%. Ce gel peut être formé de nombreux polyuréthanes gélifiants selon l'invention. Cette huile gélifiée est très fluide et vaporisable. Les nacres sont maintenues en suspension grâce au polyuréthane gélifiant et ce durant toute la stabilité du produit. Des nacres de granulométrie et de densités variables peuvent être utilisées, a des concentrations comprises entre 0.1 à 1% dans le gel.

### 3) Huile nacrée épaisse et vaporisable :

| **INCI** | % |
|---|---|
| Caprylic/capric triglyceride | 92,55 |
| **PU GSC16179 [Exemple 1]** | **6,25** |
| Synthetic Fluorphlogopite (and) Iron Oxide ; | 0,1 |
| Parfum | 0,1 |
| Caryodendron orinocense Nut Oil | 1 |

Dans cet exemple de gelée huileuse, le gel formé reste transparent malgré la teneur élevée en polyuréthane gélifiant, les nacres sont parfaitement maintenues en suspension et ce durant toute la stabilité du produit. Ce gel peut être formé de nombreux polyuréthanes gélifiants selon l'invention. Le polyuréthane gélifiant forme un gel transparent, épais, nacré et vaporisable grâce à la rhéologie thixotrope du gel formé.

### 4) Huile sèche pour le corps

| **INCI** | **%** |
|---|---|
| Isononyl isononanoate | 54,5 |
| Isopropyl palmitate | 20 |
| Dicaprylyl carbonanate | 20 |
| **PU GSC17020** | **5** |
| Tocopheryl acetate | 0,2 |
| Parfum | 0,3 |

Le polyuréthane gélifiant forme un gel transparent dans ce mélange d'huile. Le gel formé est épais, stable et vaporisable. Le polyuréthane gélifiant GSC17020 est identique au lot GSC17016 décrit dans l'exemple 2 mais avec une quantité de 1,10-décanediol de 0,65 équivalent au lieu de 0,6 équivalent.

### 5) Baume à lèvres transparent

| **INCI** | **%** |
|---|---|
| Isopropyl palmitate | 36,9 |
| Caprylic / capric triglycéride | 30 |
| Ricinus communis seed oil | 11,5 |
| Dimer dilinoleyl dimer dilinoleate | 6,4 |
| C10-18 Triglycerides | 5,8 |
| PU GSC17020 | 6,4 |
| Bis-diglyceryl polyacyladipate-2 | 3 |

Le polyuréthane gélifiant forme un gel transparent dans ce mélange d'huile et de cire. Le polyuréthane gélifiant GSC17020, réduit la cristallisation des cires et beurres ce qui permet de formuler des gels transparents pour les lèvres. Le gel formé est épais et se présente facilement dans un pot.

### 6) Gloss de faible viscosité

| INCI | % |
|---|---|
| Pentaerythrityl tetraisostearate | 30 |
| Dimer dilinoleyl dimer dilinoleate | 20 |
| Ricinus communis seed oil | 46,1 |
| Tocopheryl acetate | 0,2 |
| **PU16179 [Exemple 1]** | **2,5** |
| CI 15850, Synthetic Wax | 0,2 |
| Titanium Dioxide (and) Synthetic Fluorphlogopite (and) Iron Oxide | 1 |
| Parfum | 0,5 |

Le polyuréthane gélifiant forme un gel transparent dans ce mélange d'huile. Le polyuréthane gélifiant maintient en suspension les nacres et pigments. Le gel formé est peu épais et se présente facilement dans une bouillote à gloss. Ce gel peut être formé avec polyuréthane GSC16179 [Exemple 1] ou GSC17020. Selon l'exemple de polyuréthane gélifiant utilisé, les nacres ne sont pas maintenues en suspension.

Le polyuréthane gélifiant est sans aucun danger pour les muqueuses labiales.

### 7) Gloss de viscosité moyenne

| INCI | % |
|---|---|
| Pentaerythrityl tetraisostearate | 30 |
| Dimer dilinoleyl dimer dilinoleate | 20 |
| Ricinus communis seed oil | 46,1 |
| Tocopheryl acetate | 0,2 |
| **PU GSC16179 [Exemple 1]** | **5** |
| Cl 15850, Synthetic Wax | 0,2 |
| Titanium Dioxide (and) Synthetic Fluorphlogopite (and) Iron Oxide | 1 |
| Parfum | 0,5 |

Le polyuréthane gélifiant forme un gel transparent dans ce mélange d'huile. Le polyuréthane gélifiant maintient en suspension les nacres et pigments. Le gel formé est moyennement épais et se présente facilement dans une bouillote à gloss. Ce gel peut également être formé avec les polyuréthanes gélifiants GSC16100 ou GSC17020. Les nacres sont parfaitement maintenues en suspension durant toute la durée des tests de stabilité.

Le polyuréthane gélifiant est sans aucun danger pour les muqueuses labiales.

### 8) Gloss de viscosité élevée présentable en pot

| INCI | % |
|---|---|
| Pentaerythrityl tetraisostearate | 30 |
| Dimer dilinoleyl dimer dilinoleate | 20 |
| Ricinus communis seed oil | 38,6 |
| Tocopheryl acetate | 0,2 |
| **PU16179 [Exemple 1]** 1 | **10** |
| Cl 15850, synthetic wax | 0,2 |
| Titanium Dioxide (and) Synthetic Fluorphlogopite (and) Iron Oxide | |
| Parfum | 0,5 |

Le polyuréthane gélifiant forme un gel transparent dans ce mélange d'huile. Le polyuréthane gélifiant maintient en suspension les nacres et pigments. Le gel formé est épais et se présente facilement dans un pot. Ce gel peut également être formé avec le polyuréthane gélifiant GSC17020. Les nacres sont parfaitement maintenues en suspension durant toute la durée des tests de stabilité (plusieurs mois).

Le polyuréthane gélifiant est sans aucun danger pour les muqueuses labiales.

### 9) Exfoliant pour le corps

| **INCI** | **%** |
|---|---|
| Caprylic/capric triglycéride | 83,40 |
| **PU GSC17020** | **6,00** |
| Sucrose | 4,00 |
| Synthetic Wax (and) CI 77891 (and) CI 77510 | 1,00 |
| PEG-20 Glyceryl Triisostearate | 5,00 |
| Parfum | 0,50 |
| Diethylhexyl Syringylidenemalonate (and) Caprylic/Capric Triglycéride | 0,10 |
| Hydrogenated Palm Kernel Glycerides and Hydrogenated Palm Glycerides | 5,00 |

Le polyuréthane gélifiant permet par exemple de maintenir des exfoliants de densité variable en suspension dans un gel huileux contenant entre 5 et 10% de polyuréthane gélifiant. Le polyuréthane gélifiant maintient en suspension, par exemple, 4% de sucre ou de sel ou de la cire synthétique, ou de corindon ou de pierre ponce. Le gel de base est transparent. L'aspect thixotrope du gel permet un étalement fluide alors que le produit est très épais en pot.

### 10) Huile solaire gélifiée avec des filtres organiques

| INCI | % |
|---|---|
| Butyl Methoxydibenzoylmethane | 3 |
| Octocrylene | 8 |
| Ethylhexyl Salicylate | 5 |
| Homosalate | 10 |
| Trimethoxybenzylidene Pentanedione | 1 |
| C12-C15 Alkyl benzoate | 67,8 |
| **PU GSC16179 [Exemple 1]** | **5** |
| Parfum | 0,2 |

Le polyuréthane gélifiant forme un gel transparent dans des mélanges contenant de l'octocrylene, de l'ethylhexylsalicylate, de l'homosalate, et / ou du butylmethoxydibenzoylmethane. Le polyuréthane gélifiant est compatible et stable avec la plupart des filtres organiques. Le gel peut également être formé avec le polyuréthane gélifiant GSC17020.

### 11) Huile solaire gélifiée avec des filtres organiques et minéraux

| **INCI** | **%** |
|---|---|
| Butyl Methoxydibenzoylmethane | 1,5 |
| Octocrylene | 4 |
| Ethylhexyl Salicylate | 2,5 |
| Homosalate | 5 |
| Trimethoxybenzylidene Pentanedione | 0,5 |
| Caprylic/capric triglyceride | 50 |
| C12-C15 Alkyl benzoate | 32,4 |
| **PU GSC16179 [Exemple 1]** | **2,5** |
| Parfum | 0,1 |
| Titanium Dioxide | 1,5 |

Le polyuréthane gélifiant maintient des filtres inorganiques en suspension tels que par exemple le dioxide de titane dans un gel épais et vaporisable.

Le gel peut peut également être formé avec le polyuréthane gélifiant GSC17020.

### 12)Gelée démaquillante

| **INCI** | % |
|---|---|
| Ethylhexyl Cocoate (and) Cocos Nucifera Oil | 20 |
| C12-C15 alkyl benzoate | 20 |
| Cocos nucifera oil (and) hydrogenated coconut oil | 3 |
| Caprylic/Capric Triglycéride | 41,3 |
| **PU GSC17020** | **5** |
| PEG-40 Sorbitan Peroleate | 10 |
| Tocopheryl Acetate | 0,2 |
| Parfum | 0,5 |

Le polyuréthane gélifiant permet de former des gels dans des huiles nettoyantes avec une grande teneur en émulsionnant. Le polyuréthane gélifiant est compatible avec les émulsionnants huileux.

Le gel formé est épais, totalement transparent et stable.

### 13) Emulsion eau dans huile

| INCI | % |
|---|---|
| Water Aqua | 64,4 |
| Magnésium sulfate | 0,7 |
| Glycerin | 5 |
| Phenoxyethanol (and) ethylhexylglycerin | 0,8 |
| Propanediol | 1 |
| Octyldodecanol (and) octyldecyl xyloside (and) PEG-30 dipolyhydroxystearate | 4 |
| Caprylic/capric triglycéride | 21,6 |
| **PU GSC16179 [Exemple 1]** | **2,4** |
| Parfum (fragrance) | 0,1 |

Le polyuréthane gélifiant s'incorpore facilement dans les formules émulsion eau dans huile et augmente la viscosité. Cette émulsion peut également être formée avec le polyuréthane gélifiant GSC17020.

### 14)Crème de nuit réparatrice

| **INCI** | % |
|---|---|
| Butyrospermum parkii Butter | 5 |
| Polyglyceryl-3 diisostearate | 4 |
| Dicaprylyl carbonate | 15 |
| **PU GSC16179 [Exemple 1]** | **5** |
| Water / Aqua | 57 |
| Magnésium sulfate | 0,7 |
| Glycerin | 10 |
| 1 ,2-Hexanediol (And) Caprylyl Glycol | 0,5 |
| Water (And) Alcohol (And) Onopordum Acanthium Flower/Leaf/Stem Extract | 2 |
| Perfume | 0,5 |
| Tocopheryl acetate | 0,3 |

Cette crème de nuit réparatrice ne présente pas d'instabilité lorsqu'il contient le polyuréthane gélifiant alors que la formule qui n'en contient pas est instable. Cette émulsion peut également être formée avec le polyuréthane gélifiant GSC17020.

### 15)Crème solaire

| **INCI** | **%** |
|---|---|
| Caprylic/Capric Triglycéride | 12,85 |
| **PU GSC17020** | **5** |
| Octyldodecanol (and) Octyldodecyl Xyloside (and) PEG-30 Dipolyhydroxystearate | 4 |
| C12-C15 Alkyl Benzoate | 12,85 |
| Sodium Chloride | 0,5 |
| Aqua | 44,5 |
| Glycerin | 5 |
| Phenoxyethanol (and) Ethylhexylglycerin | 0,5 |
| Zinc Oxide (and) Caprylic/Capric Triglycéride (and) Polyhydroxystearic Acid (and) Polyglyceryl-3 Polyricinoleate (and) Isostearic Acid (and) Lecithin | 14,3 |
| Parfum | 0,5 |

Le polyuréthane gélifiant permet d'augmenter la viscosité et de stabiliser des émulsions solaires contenant des filtres minéraux. Cet exemple ne présente pas d'instabilité lorsqu'il contient le polyuréthane gélifiant alors que la formule qui n'en contient pas est instable.

### 16) Formule huile dans eau

| **INCI** | **%** |
|---|---|
| Aqua Water | 30,9 |
| Glycerin | 30 |
| Ethylhexylglycerin & Phenoxyethanol | 1 |
| Cetearyl Alcohol | 10 |
| Cetyl alcohol (and) glyceryl stéarate (and) PEG-75 stéarate (and) ceteth-20 (and) stearath-20 | 2,5 |
| Caprylic/Capric Triglyceride | 20 |
| Sodium Stearoyl Glutamate | 0,5 |
| **PU GSC17020** | **5** |
| Parfum | 0,1 |

Cet exemple de formule montre le polyuréthane gélifiant dans une formule à phase continue aqueuse. Le polyuréthane gélifiant s'incorpore correctement dans le mélange et la formule est stable.

### 17) Emulsion gel crème

| **INCI** | **%** |
|---|---|
| Eau | 73 |
| Acrylate / C10 C30 Alkyl acrylate crosspolymer | 0,6 |
| Tetrasodium EDTA | 0,05 |
| Ethylhexylglycerin & Phenoxyethanol | 0,8 |
| Caprylic/Capric Triglycéride | 20 |
| **PU GSC17020** | **5** |
| Triethanolamine | 0,55 |

Cet exemple de formule montre le polyuréthane gélifiant dans une formule à phase continue aqueuse sans émulsionnant. Le polyuréthane gélifiant s'incorpore correctement dans le mélange et la formule est stable.

### 18) Emulsion sans eau

| **INCI** | **%** |
|---|---|
| Propylen glycol | 30,40 |
| Glycerin | 30,00 |
| Sodium Polyacrylate | 0,10 |
| Cetearyl Alcohol | 2,00 |
| Cetyl alcohol (and) glyceryl stéarate (and) PEG-75 stéarate (and) ceteth-20 (and) stearath-20 | 2,00 |
| Sodium Stearoyl Glutamate | 0,50 |
| Caprylic/Capric Triglyceride | 31,00 |
| **PUGSC17020** | 4,00 |

Cet exemple de formule montre le polyuréthane gélifiant dans une formule à phase continue glycol. Le polyuréthane gélifiant s'incorpore correctement dans le mélange et la formule est plus stable avec le polyuréthane gélifiant que sans le polyuréthane gélifiant où elle déphase totalement.

### 19) Mousse huile dans eau

| **INCI** | % |
|---|---|
| Caprylic/capric triglycérides | 23 |
| **PU GSC16179 (Exemple 1)** | 2 |
| Polyglyceryl-6 Distearate (and) Jojoba Esters (and) Polyglyceryl-3 Beeswax (and) Cetyl Alcohol | 3 |
| Hydrogenated Palm Kernel Glycerides and Hydrogenated Palm Glycerides | 2 |
| Water / Aqua | 64,15 |
| Phenoxyethanol (and) ethylhexylglycerin | 0,8 |
| Propanediol | 2 |
| TETRASODIUM EDTA | 0,05 |
| POLOXAMER 407 (and) PPG-12/SMDI COPOLYMER | 3 |

Le polyuréthane gélifiant peut être incorporé dans des émulsions huile dans eau type mousse foisonnée. Le polyuréthane gélifiant s'incorpore correctement dans le mélange et la formule est stable.

### 20) Formule de mascara waterproof

| **INCI** | **%** |
|---|---|
| Water / Aqua | 55 |
| Magnésium sulfate | 0,63 |
| Glycerin | 5 |
| Phenoxyethanol (and) ethylhexylglycerin | 0,8 |
| Propanediol | 1 |
| Octyldodecanol (and) octyldecyl xyloside (and) PEG-30 dipolyhydroxystearate | 4 |
| Caprylic/capric triglycéride | 19,57 |
| **PU GSC16179 (Exemple 1)** | 2,55 |
| C10-18 triglycérides | 3 |
| Iron oxides, Triethoxycaprylylsilane, Isononyl Isononanoate, Ethylene/Propylene/Styrene Copolymer, Sorbitan Oleate | 6,5 |
| ozokerite | 2 |

Le polyuréthane gélifiant permet de formuler des émulsions fortement teintées telle qu'un mascara. Le polyuréthane gélifiant augmente la viscosité du mélange, stabilise les réseaux cireux, améliore l'accroche sur la brosse et la dépose sur le cil. Le polyuréthane gélifiant s'incorpore correctement dans le mélange et la formule est stable.

### Exemple 13 : Mesure de la viscosité du polyuréthane gélifiant de l'exemple 2 selon l'invention et du polyuréthane de l'exemple 5 de la demande WO2016/090081 dans du triglycéride caprylique / caprique (exemple comparatif).

Le composé de l'exemple 5 de la demande WO2016/090081 a été préparé comme décrit.

Les mesures de viscosité ont été réalisées sur un gel à 5% en masse de chacun des polyuréthanes dans les conditions décrites dans la demande WO2016/090081 avec un viscosimètre TA Instruments AR1500ex, par mesure de rhéologie en écoulement, à un taux de cisaillement de 1 s⁻¹ et à 20 °C.

Les résultats sont rapportés dans le tableau 6 ci-dessous.

| Exemple | Numéro de lot | Viscosité en Pa.s | Aspect |
|---|---|---|---|
| 2 | GSC17016 | 53,6 | transparent |
| 5 demande WO2016/090081 (exemple comparatif) | - | 1,7 | translucide |

Les résultats montrent que le gel contenant le polyuréthane de l'exemple 2 selon l'invention présente des propriétés totalement différentes de celles du gel contenant le polyuréthane de l'exemple 5 de la demande WO2016/090081, à savoir une viscosité significativement supérieure et un aspect transparent.

## Revendications

1. Composé polyuréthane apte à gélifier des huiles, de formule (I) 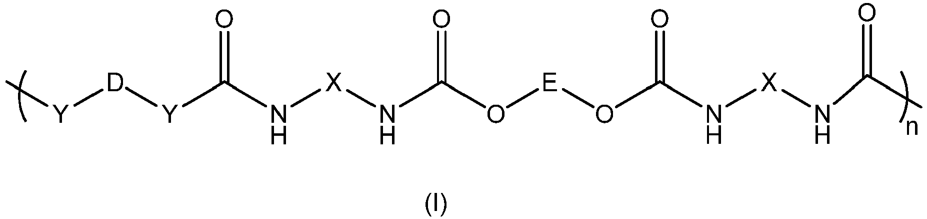 dans laquelle 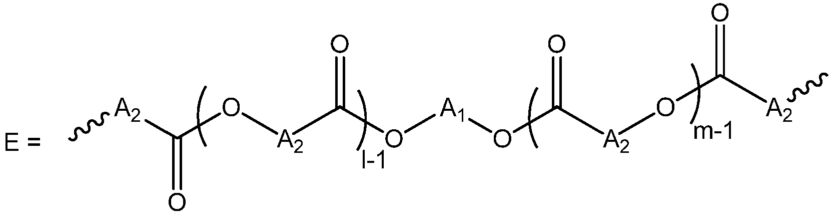
- A₁ représente un radical alkyl, linéaire ou ramifié, comprenant de 2 à 20 atomes de carbone, de préférence de 2 à 12, et en particulier de 2 à 6, ledit radical comprenant éventuellement une ou plusieurs insaturations, étant éventuellement interrompu par au moins un hétéroatome choisi parmi O, N et S, et étant éventuellement substitué par au moins un substituant -OAlk, Alk représentant un groupe alkyle comprenant de 1 à 10 atomes de carbone
- A₂ représente un radical alkyl, linéaire ou ramifié, comprenant de 2 à 20 atomes de carbone, de préférence de 5 à 18, et préférentiellement de 6 à 17, ledit radical comprenant éventuellement une ou plusieurs insaturations, et étant éventuellement substitué par au moins un substituant -OAlk, Alk représentant un groupe alkyle comprenant de 1 à 10 atomes de carbone
- l et m représentent, indépendamment l'un de l'autre, un nombre entier de 1 à 20, de préférence de 1 à 10, la somme l+m étant, de préférence, de 2 à 20.
- n est un nombre entier de 1 à 20, de préférence de 2 à 8,
- D représente un radical alkyl, linéaire ou ramifié, comprenant de 2 à 40 atomes de carbone, de préférence 6 à 14 atomes de carbone, ledit radical comprenant éventuellement une ou plusieurs insaturations,
- X est un radical alkyl linéaire ou ramifié, présentant ou non des insaturations, en C₂-C₂₀,
- Y représente un atome ou un groupe choisi parmi O, NH, S et C(O)O.

2. Composé polyuréthane de formule (I) selon la revendication 1, dans laquelle au moins une des conditions suivantes est remplie :
- A₁ représente un radical alkyl linéaire comprenant de 2 à 12, de préférence 2 à 6, en particulier 3 atomes de carbone, ledit radical comprenant éventuellement une ou plusieurs insaturations ;
- A₂ représente un radical alkyl, linéaire ou ramifié, comprenant de 2 à 20 atomes de carbone, notamment de 12 à 18 atomes de carbone, ledit radical comprenant éventuellement une ou plusieurs insaturations, et de manière plus préférée un groupe de formule 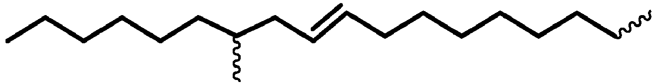
- l et m représentent, indépendamment un nombre entier de 1 à 10, la somme l+m étant, de préférence, de 2 à 20 ;
- la somme l+m a une valeur moyenne de 5 à 12, en particulier 7 ou 11,
- n est un nombre entier de 2 à 8 ;
- D est un radical alkyl, linéaire ou ramifié, comprenant de 6 à 14 atomes de carbone, ledit radical comprenant éventuellement une ou plusieurs insaturations
- X est un radical alkyl linéaire ou ramifié, présentant ou non des insaturations, comprenant de 2 à 10, en particulier 6 atomes de carbone.
- Y représente un atome d'oxygène.

3. Composé polyuréthane de formule (I) selon la revendication 1, dans laquelle
- A₁ représente un radical alkyl, linéaire ou ramifié, comprenant de 2 à 6, en particulier 3 atomes de carbone ;
- A₂ représente un radical alkyl, linéaire ou ramifié, comprenant de 12 à 18 atomes de carbone, ledit radical comprenant éventuellement une ou plusieurs insaturations ;
- l et m représentent, indépendamment l'un de l'autre, un nombre entier de 1 à 10, la somme l+m étant, de préférence, de 5 à 12 ;
- n est un nombre entier de 2 à 8 ;
- D représente un radical alkyl, linéaire ou ramifié, comprenant 6 à 14 atomes de carbone, ledit radical comprenant éventuellement une ou plusieurs insaturations ;
- X est un radical alkyl linéaire ou ramifié, présentant ou non des insaturations, comprenant de 2 à 10, en particulier 6 atomes de carbone, et
- Y représente un atome d'oxygène.

4. Composé polyuréthane de formule (I) selon l'une des revendications 1 à 3 dans lequel A₁ représente un radical alkyl linéaire comprenant 3 atomes de carbone et/ou . A₂ représente un groupe de formule

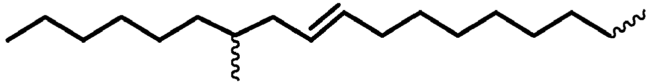

5. Composé polyuréthane de formule (I) selon l'une des revendications 1 à 4 présentant un contenu d'origine naturelle à plus de 80% selon la norme NF-16128-2.

6. Procédé de préparation d'un composé polyuréthane de formule (I) 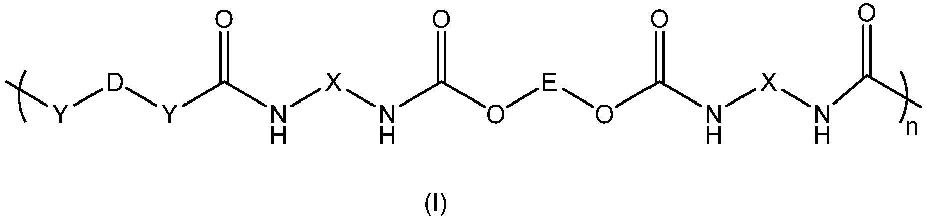 dans laquelle 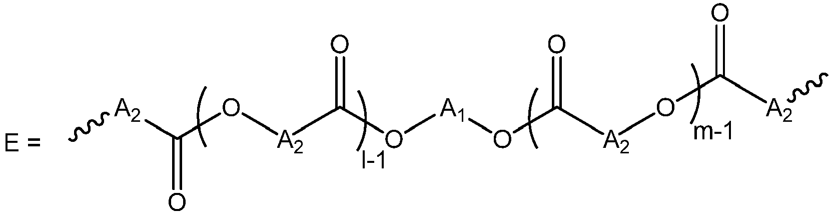
- A₁ représente un radical alkyl, linéaire ou ramifié, comprenant de 2 à 20 atomes de carbone, de préférence de 2 à 12, et en particulier de 2 à 6, ledit radical comprenant éventuellement une ou plusieurs insaturations, étant éventuellement interrompu par au moins un hétéroatome choisi parmi O, N et S, et étant éventuellement substitué par au moins un substituant -OAlk, Alk représentant un groupe alkyle comprenant de 1 à 10 atomes de carbone
- A₂ représente un radical alkyl, linéaire ou ramifié, comprenant de 2 à 20 atomes de carbone, de préférence de 5 à 18, et préférentiellement de 6 à 17, ledit radical comprenant éventuellement une ou plusieurs insaturations, et étant éventuellement substitué par au moins un substituant -OAlk, Alk représentant un groupe alkyle comprenant de 1 à 10 atomes de carbone
- l et m représentent, indépendamment l'un de l'autre, un nombre entier de 1 à 20, de préférence de 1 à 10, la somme l+m étant, de préférence, de 2 à 20.
- n est un nombre entier de 1 à 20, de préférence 2 à 8,
- D représente un radical alkyl, linéaire ou ramifié, comprenant de 2 à 40 atomes de carbone, de préférence 6 à 14 atomes de carbone, ledit radical comprenant éventuellement une ou plusieurs insaturations,
- X est un radical alkyl linéaire ou ramifié, présentant ou non des insaturations, en C₂-C₂₀,
- Y représente un atome ou un groupe choisi parmi O, NH, S et C(O)O, comprenant les étapes suivantes :
1) La fonctionnalisation d'un estolide di-OH de formule (3) par un dérivé diisocyanate de formule (4) pour obtenir un estolide diisocyanate de formule (5) 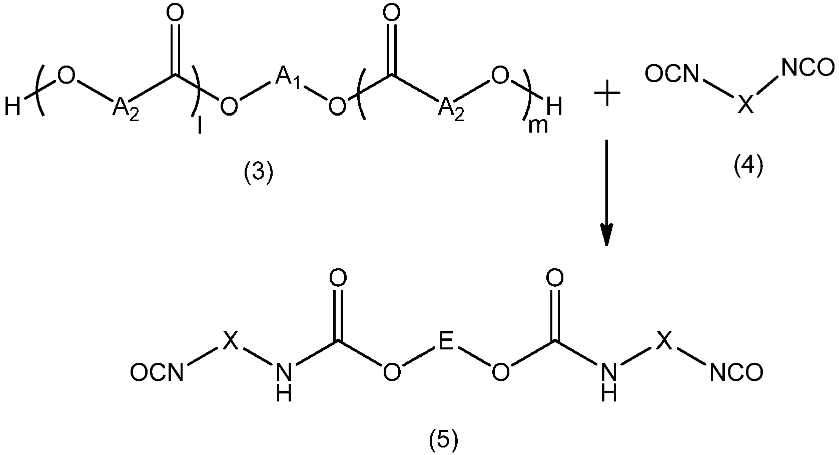 dans lesquelles : 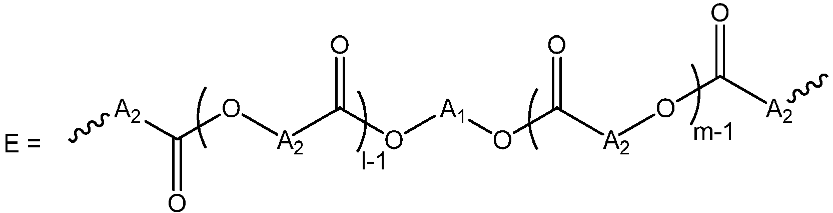
- A₁, A₂, l et m sont tels que définis ci-dessus ;
- X est un radical alkyl linéaire ou ramifié, présentant ou non des insaturations, en C₂-C₂₀,
2) L'extension de chaine pour obtenir un polyuréthane d'estolide de formule (7) par ajout d'un diol de formule (6), optionnellement solubilisé dans une huile 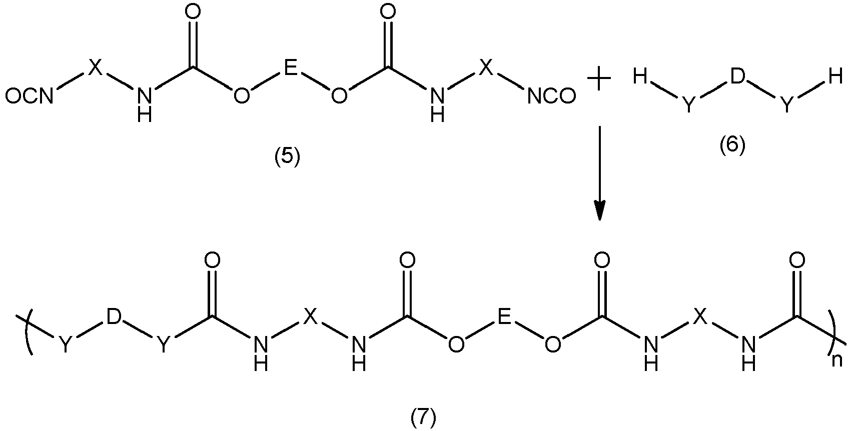 dans lesquelles
- A₁, A₂, E, X, Y, l et m sont tels que définis ci-dessus ;
- D représente un radical alkyl, linéaire ou ramifié, comprenant de 2 à 40 atomes de carbone, de préférence de 6 à 14 atomes de carbone, ledit radical comprenant éventuellement une ou plusieurs insaturations;
- n est un nombre entier de 1 à 20, de préférence 2 à 8 ;
et
3) optionnellement, la terminaison de chaine par ajout d'une molécule nucléophile en excès pouvant réagir avec les fonctions isocyanates résiduelles pour donner le polyuréthane gélifiant de formule (I).

7. Procédé selon la revendication 6, dans lequel au moins une des conditions suivantes est remplie :
- A₁ représente un radical alkyl linéaire comprenant de 2 à 12, de préférence 2 à 6, en particulier 3 atomes de carbone, ledit radical comprenant éventuellement une ou plusieurs insaturations ;
- A₂ représente un radical alkyl, linéaire ou ramifié, comprenant de 2 à 20 atomes de carbone, notamment de 12 à 18 atomes de carbone, ledit radical comprenant éventuellement une ou plusieurs insaturations, et de manière plus préférée un groupe de formule 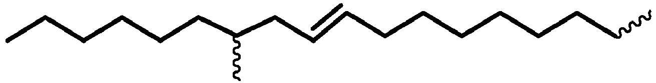
- l et m représentent, indépendamment un nombre entier de 1 à 10, la somme l+m étant, de préférence, de 2 à 20 ;
- la somme l+m a une valeur moyenne de 5 à 12, en particulier 7 ou 11,
- n est un nombre entier de 2 à 8 ;
- D est un radical alkyl, linéaire ou ramifié, comprenant de 6 à 14 atomes de carbone, ledit radical comprenant éventuellement une ou plusieurs insaturations
- X est un radical alkyl linéaire ou ramifié, présentant ou non des insaturations, comprenant de 2 à 10, en particulier 6 atomes de carbone.
- Y représente un atome d'oxygène.

8. Procédé l'une des revendications 6 ou 7 dans lequel A₁ représente un radical alkyl linéaire comprenant 3 atomes de carbone, et/ou dans lequel A₂ représente un groupe de formule

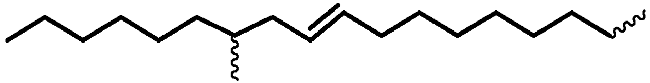

9. Procédé selon l'une des revendications 6 à 8, dans lequel l'étape de fonctionnalisation de l'estolide di-OH par un dérivé diisocyanate est effectuée :
- par ajout rapide d'une quantité de diisocyanate (4) comprise entre 1,4 et 2,0 équivalents par rapport à 1 équivalent (eq) d'estolide di-OH (3), calculé d'après son indice d'OH, à une température comprise entre 60°C et 150°C, de préférence entre 110°C et 135°C, ou
- en ajoutant 1 équivalent (eq) d'estolide di-OH (3) sur un mélange d'huile et de diisocyanate (4) présent en quantité comprise entre 1,4 à 2,0 équivalents, à une température supérieure ou égale à 110°C.

10. Procédé selon l'une quelconque des revendications 6 à 9, dans lequel la terminaison de chaine est effectuée par l'ajout en excès d'une molécule nucléophile de type alcool, thiol, amine ou carboxylate, mono- ou difonctionnelle, pouvant réagir avec les fonctions isocyanates résiduelles en excès au milieu réactionnel à une température comprise entre 110 et 150°C.

11. Utilisation du polyuréthane gélifiant de formule (I) selon l'une quelconque des revendications 1 à 5, ou obtenu selon le procédé de l'une quelconque des revendications 6 à 10, comme agent gélifiant d'huiles.

12. Procédé de gélification d'au moins une huile choisie, par exemple, parmi les huiles organiques, dont les huiles végétales, et les mélanges d'huiles apolaires et d'huiles plus polaires, dans lequel un polyuréthane gélifiant de formule (I) selon l'une quelconque des revendications 1 à 5, ou obtenu selon le procédé de l'une quelconque des revendications 6 à 10, est ajouté à ladite ou auxdites huile(s), ou à une composition la ou les contenant.

13. Gel contenant ou formé à partir du polyuréthane gélifiant de formule (I) selon l'une quelconque des revendications 1 à 5, ou du polyuréthane gélifiant de formule (I) obtenu selon le procédé de l'une quelconque des revendications 6 à 10.

14. Gel selon la revendication 13, dans lequel la teneur massique du polyuréthane gélifiant de formule (I) est comprise entre 0,7% et 10%, de préférence de 1 à 5%.

15. Composition pharmaceutique ou cosmétique comprenant un gel selon l'une des revendications 13 ou 14.

## Patentansprüche

1. Polyurethanverbindung, die zum Gelieren von Ölen fähig ist, der Formel (I): 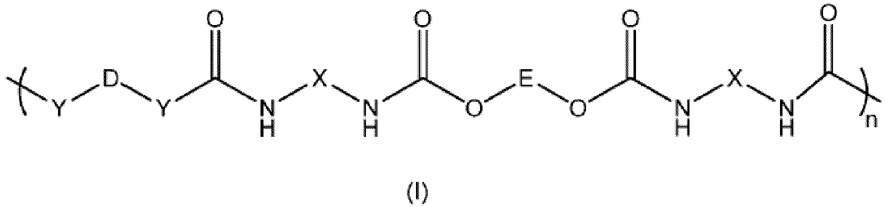 wobei 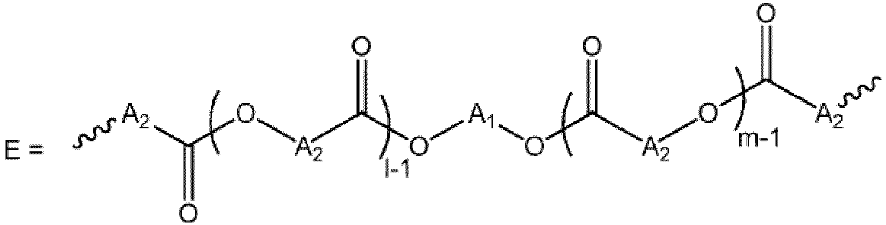
- A₁ für einen Alkylrest, linear oder verzweigt, umfassend 2 bis 20 Kohlenstoffatome, vorzugsweise 2 bis 12 und insbesondere 2 bis 6, steht, wobei der Rest gegebenenfalls eine oder mehrere Ungesättigtheiten umfasst, gegebenenfalls durch mindestens ein Heteroatom unterbrochen ist, das aus O, N und S ausgewählt ist, und gegebenenfalls durch mindestens einen Substituenten -OAlk substituiert ist, wobei Alk für eine Alkylgruppe, umfassend 1 bis 10 Kohlenstoffatome, steht,
- A₂ für einen Alkylrest, linear oder verzweigt, umfassend 2 bis 20 Kohlenstoffatome, vorzugsweise 5 bis 18 und präferentiell 6 bis 17, steht, wobei der Rest gegebenenfalls eine oder mehrere Ungesättigtheiten umfasst und gegebenenfalls durch mindestens einen Substituenten - OAlk substituiert ist, wobei Alk für eine Alkylgruppe, umfassend 1 bis 10 Kohlenstoffatome, steht,
- 1 und m unabhängig voneinander für eine ganze Zahl von 1 bis 20, vorzugsweise 1 bis 10 stehen, wobei die Summe 1 + m vorzugsweise 2 bis 20 beträgt,
- n eine ganze Zahl von 1 bis 20, vorzugsweise 2 bis 8 ist,
- D für einen Alkylrest, linear oder verzweigt, umfassend 2 bis 40 Kohlenstoffatome, vorzugsweise 6 bis 14 Kohlenstoffatome, steht, wobei der Rest gegebenenfalls eine oder mehrere Ungesättigtheiten umfasst;
- X ein C₂-C₂₀-Alkylrest, linear oder verzweigt, aufweisend Ungesättigtheiten oder auch nicht, ist,
- Y für ein Atom oder eine Gruppe steht, das bzw. die aus O, NH, S und C(O)O ausgewählt ist.

2. Polyurethanverbindung der Formel (I) nach Anspruch 1, wobei mindestens eine der folgenden Bedingungen erfüllt ist:
- A₁ steht für einen linearen Alkylrest, umfassend 2 bis 12, vorzugsweise 2 bis 6, insbesondere 3 Kohlenstoffatome, wobei der Rest gegebenenfalls eine oder mehrere Ungesättigtheiten umfasst;
- A₂ steht für einen Alkylrest, linear oder verzweigt, umfassend 2 bis 20 Kohlenstoffatome, insbesondere 12 bis 18 Kohlenstoffatome, wobei der Rest gegebenenfalls eine oder mehrere Ungesättigtheiten und besonders bevorzugt eine Gruppe der Formel umfasst: 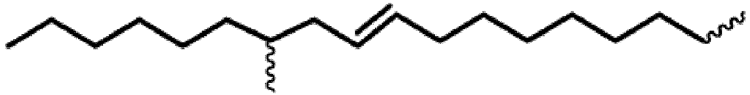
- 1 und m stehen unabhängig für eine ganze Zahl von 1 bis 10, wobei die Summe 1 + m vorzugsweise 2 bis 20 beträgt;
- die Summe 1 + m weist einen durchschnittlichen Wert von 5 bis 12, insbesondere 7 oder 11 auf,
- n ist eine ganze Zahl von 2 bis 8;
- D ist ein Alkylrest, linear oder verzweigt, umfassend 6 bis 14 Kohlenstoffatome, wobei der Rest gegebenenfalls eine oder mehrere Ungesättigtheiten umfasst;
- X ist ein Alkylrest, linear oder verzweigt, aufweisend Ungesättigtheiten oder auch nicht, umfassend 2 bis 10, insbesondere 6 Kohlenstoffatome;
- Y steht für ein Sauerstoffatom.

3. Polyurethanverbindung der Formel (I) nach Anspruch 1, wobei
- A₁ für einen linearen Alkylrest, linear oder verzweigt, umfassend 2 bis 6, insbesondere 3 Kohlenstoffatome, steht;
- A₂ für einen Alkylrest, linear oder verzweigt, umfassend 12 bis 18 Kohlenstoffatome, steht, wobei der Rest gegebenenfalls eine oder mehrere Ungesättigtheiten umfasst;
- 1 und m unabhängig voneinander für eine ganze Zahl von 1 bis 10 stehen, wobei die Summe 1 + m vorzugsweise 5 bis 12 beträgt;
- n eine ganze Zahl von 2 bis 8 ist;
- D für einen Alkylrest, linear oder verzweigt, umfassend 6 bis 14 Kohlenstoffatome, steht, wobei der Rest gegebenenfalls eine oder mehrere Ungesättigtheiten umfasst;
- X ein Alkylrest, linear oder verzweigt, aufweisend Ungesättigtheiten oder auch nicht, umfassend 2 bis 10, insbesondere 6 Kohlenstoffatome, ist, und
- Y für ein Sauerstoffatom steht.

4. Polyurethanverbindung der Formel (I) nach einem der Ansprüche 1 bis 3, wobei A₁ für einen linearen Alkylrest, umfassend 3 Kohlenstoffatome, steht und/oder A₂ für eine Gruppe der Formel steht:

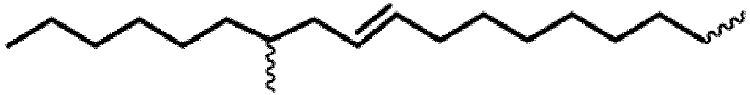

5. Polyurethanverbindung der Formel (I) nach einem der Ansprüche 1 bis 4, aufweisend einen Inhalt natürlichen Ursprungs von mehr als 80 % gemäß der Norm NF-16128-2.

6. Verfahren zur Herstellung einer Polyurethanverbindung der Formel (I): 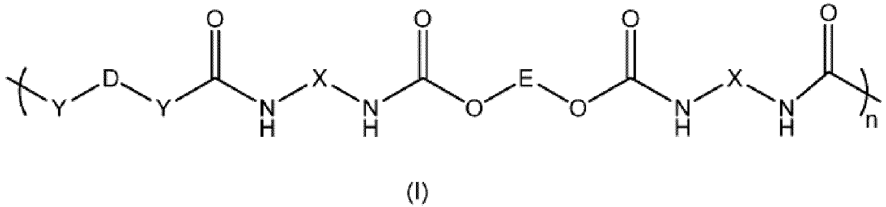 wobei 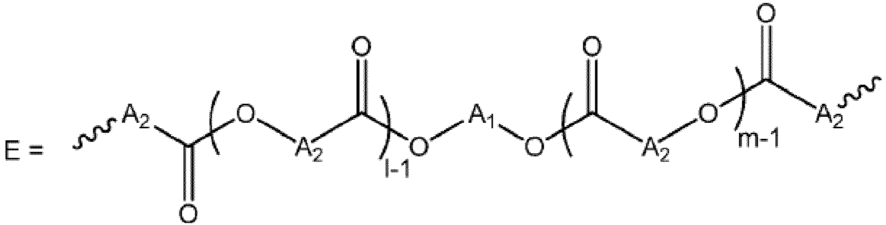
- A₁ für einen Alkylrest, linear oder verzweigt, umfassend 2 bis 20 Kohlenstoffatome, vorzugsweise 2 bis 12 und insbesondere 2 bis 6, steht, wobei der Rest gegebenenfalls eine oder mehrere Ungesättigtheiten umfasst, gegebenenfalls durch mindestens ein Heteroatom unterbrochen ist, das aus O, N und S ausgewählt ist, und gegebenenfalls durch mindestens einen Substituenten -OAlk substituiert ist, wobei Alk für eine Alkylgruppe, umfassend 1 bis 10 Kohlenstoffatome, steht,
- A₂ für einen Alkylrest, linear oder verzweigt, umfassend 2 bis 20 Kohlenstoffatome, vorzugsweise 5 bis 18 und präferentiell 6 bis 17, steht, wobei der Rest gegebenenfalls eine oder mehrere Ungesättigtheiten umfasst und gegebenenfalls durch mindestens einen Substituenten - OAlk substituiert ist, wobei Alk für eine Alkylgruppe, umfassend 1 bis 10 Kohlenstoffatome, steht,
- 1 und m unabhängig voneinander für eine ganze Zahl von 1 bis 20, vorzugsweise 1 bis 10 stehen, wobei die Summe 1 + m vorzugsweise 2 bis 20 beträgt,
- n eine ganze Zahl von 1 bis 20, vorzugsweise 2 bis 8 ist,
- D für einen Alkylrest, linear oder verzweigt, umfassend 2 bis 40 Kohlenstoffatome, vorzugsweise 6 bis 14 Kohlenstoffatome, steht, wobei der Rest gegebenenfalls eine oder mehrere Ungesättigtheiten umfasst,
- X ein C₂-C₂₀-Alkylrest, linear oder verzweigt, aufweisend Ungesättigtheiten oder auch nicht, ist,
- Y für ein Atom oder eine Gruppe steht, das bzw. die aus O, NH, S und C(O)O ausgewählt ist, umfassend die folgenden Schritte:
1) Funktionalisierung eines Di-OH-estolids der Formel (3) durch ein Diisocyanatderivat der Formel (4), um ein Diisocyanatestolid der Formel (5) zu erhalten: 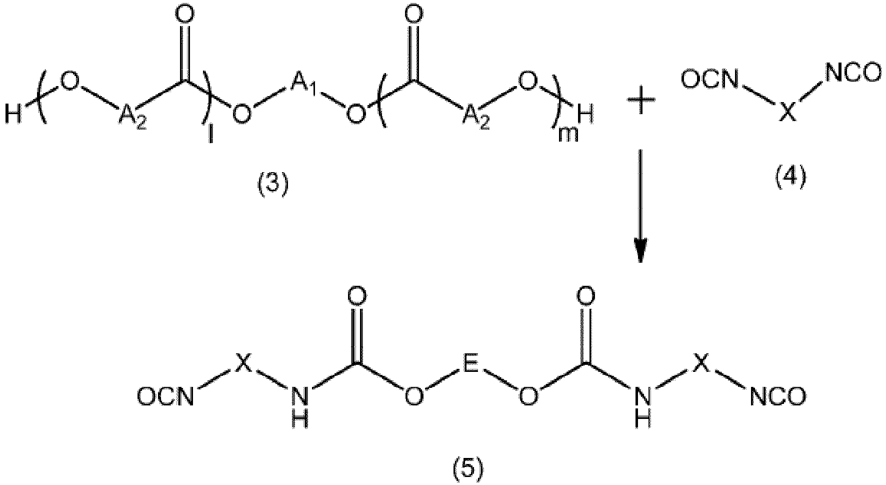 wobei: 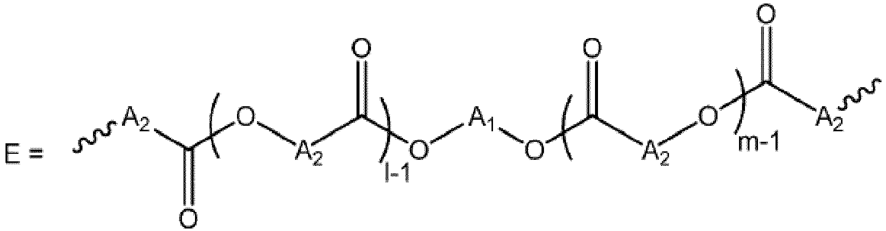
- A₁, A₂, 1 und m wie oben definiert sind;
- X ein C₂-C₂₀-Alkylrest, linear oder verzweigt, aufweisend Ungesättigtheiten oder auch nicht, ist;
2) Kettenverlängerung, um ein Estolidpolyurethan der Formel (7) zu erhalten, durch Zugabe eines Diols der Formel (6), gegebenenfalls in einem Öl solubilisiert: 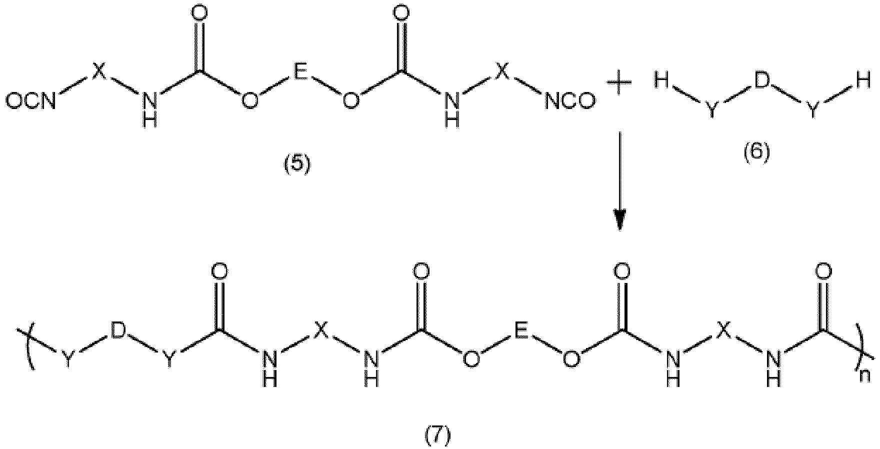 wobei
- A₁, A₂, E, X, Y, 1 und m wie oben definiert sind;
- D für einen Alkylrest, linear oder verzweigt, umfassend 2 bis 40 Kohlenstoffatome, vorzugsweise 6 bis 14 Kohlenstoffatome, steht, wobei der Rest gegebenenfalls eine oder mehrere Ungesättigtheiten umfasst;
- n eine ganze Zahl von 1 bis 20, vorzugsweise 2 bis 8 ist;
und
3) gegebenenfalls Kettentermination durch Zugabe eines nukleophilen Moleküls im Überschuss, das mit den restlichen Isocyanatfunktionen reagieren kann, um das gelierende Polyurethan der Formel (I) zu ergeben.

7. Verfahren nach Anspruch 6, wobei mindestens eine der folgenden Bedingungen erfüllt ist:
- A₁ steht für einen linearen Alkylrest, umfassend 2 bis 12, vorzugsweise 2 bis 6, insbesondere 3 Kohlenstoffatome, wobei der Rest gegebenenfalls eine oder mehrere Ungesättigtheiten umfasst;
- A₂ steht für einen Alkylrest, linear oder verzweigt, umfassend 2 bis 20 Kohlenstoffatome, insbesondere 12 bis 18 Kohlenstoffatome, wobei der Rest gegebenenfalls eine oder mehrere Ungesättigtheiten und besonders bevorzugt eine Gruppe der Formel umfasst: 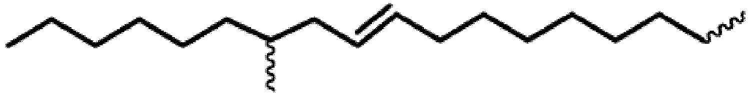
- 1 und m stehen unabhängig für eine ganze Zahl von 1 bis 10, wobei die Summe 1 + m vorzugsweise 2 bis 20 beträgt;
- die Summe 1 + m weist einen durchschnittlichen Wert von 5 bis 12, insbesondere 7 oder 11 auf,
- n ist eine ganze Zahl von 2 bis 8;
- D ist ein Alkylrest, linear oder verzweigt, umfassend 6 bis 14 Kohlenstoffatome, wobei der Rest gegebenenfalls eine oder mehrere Ungesättigtheiten umfasst;
- X ist ein Alkylrest, linear oder verzweigt, aufweisend Ungesättigtheiten oder auch nicht, umfassend 2 bis 10, insbesondere 6 Kohlenstoffatome;
- Y steht für ein Sauerstoffatom.

8. Verfahren nach einem der Ansprüche 6 oder 7, wobei A₁ für einen linearen Alkylrest, umfassend 3 Kohlenstoffatome, steht und/oder A₂ für eine Gruppe der Formel steht:

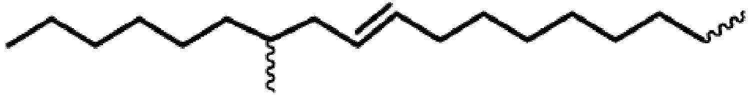

9. Verfahren nach einem der Ansprüche 6 bis 8, wobei der Schritt der Funktionalisierung des Di-OH-estolids durch ein Diisocyanatderivat durchgeführt wird:
- durch schnelle Zugabe einer Menge eines Diisocyanats (4), umfassend zwischen 1,4 und 2,0 Äquivalente bezogen auf 1 Äquivalent (Äquiv.) Di-OH-estolid (3), berechnet nach seinem OH-Index, bei einer Temperatur, die zwischen 60 °C und 150 °C, vorzugsweise zwischen 110 °C und 135 °C liegt, oder
- indem 1 Äquivalent (Äquiv.) Di-OH-estolid (3) auf ein Gemisch von Öl und Diisocyanat (4), das in einer Menge vorliegt, die zwischen 1,4 und 2,0 Äquivalenten liegt, bei einer Temperatur größer als oder gleich 110 °C zugegeben wird.

10. Verfahren nach einem der Ansprüche 6 bis 9, wobei die Kettentermination durch Zugabe eines nukleophilen Moleküls vom Alkohol-, Thiol-, Amin- oder Carboxylat-Typ, mono- oder difunktionell, im Überschuss, um mit den restlichen Isocyanatfunktionen im Überschuss im Reaktionsmedium bei einer Temperatur, die zwischen 110 und 150 °C liegt, zu reagieren, durchgeführt wird.

11. Verwendung des gelierenden Polyurethans der Formel (I) nach einem der Ansprüche 1 bis 5 oder erhalten gemäß dem Verfahren nach einem der Ansprüche 6 bis 10 als Geliermittel für Öle.

12. Verfahren zur Gelierung mindestens eines Öls, ausgewählt beispielsweise aus organischen Ölen, darunter pflanzliche Öle, und Gemischen von apolaren Ölen und stärker polaren Ölen, wobei ein gelierendes Polyurethan der Formel (I) nach einem der Ansprüche 1 bis 5 oder erhalten gemäß dem Verfahren nach einem der Ansprüche 6 bis 10 zu dem oder den Ölen oder zu einer Zusammensetzung, die dieses oder diese enthält, zugegeben wird.

13. Gel, das das gelierende Polyurethan der Formel (I) nach einem der Ansprüche 1 bis 5 oder das gemäß dem Verfahren nach einem der Ansprüche 6 bis 10 erhaltene gelierende Polyurethan der Formel (I) enthält oder zum Teil davon gebildet wird.

14. Gel nach Anspruch 13, wobei der Massengehalt des gelierenden Polyurethans der Formel (I) zwischen 0,7 % und 10 %, vorzugsweise 1 bis 5 % liegt.

15. Pharmazeutische oder kosmetische Zusammensetzung, umfassend ein Gel nach einem der Ansprüche 13 oder 14.

## Claims

1. Polyurethane compound suitable for gelling oils, of formula (I) 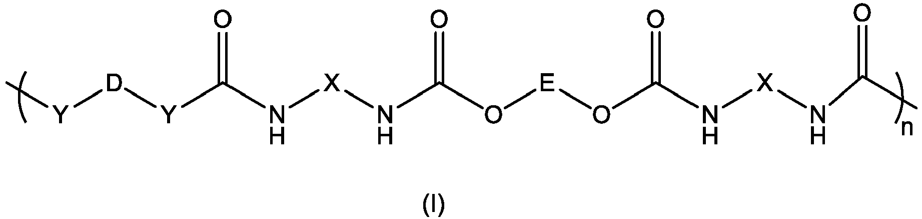 in which 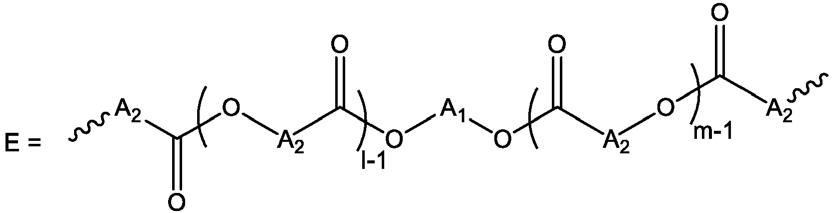
- A₁ represents a linear or branched alkyl radical, comprising from 2 to 20 carbon atoms, preferably from 2 to 12, and in particular from 2 to 6, said radical optionally comprising one or more unsaturations, optionally being interrupted by at least one heteroatom selected from O, N and S, and optionally being substituted by at least one substituent -OAlk, Alk representing an alkyl group comprising from 1 to 10 carbon atoms
- A₂ represents a linear or branched alkyl radical, comprising from 2 to 20 carbon atoms, preferably from 5 to 18, and preferentially from 6 to 17, said radical optionally comprising one or more unsaturations, and optionally being substituted by at least one substituent -OAlk, Alk representing an alkyl group comprising from 1 to 10 carbon atoms
- I and m represent, independently of one another, an integer from 1 to 20, preferably from 1 to 10, the sum of I+m being, preferably, from 2 to 20.
- n is an integer from 1 to 20, preferably from 2 to 8,
- D represents a linear or branched alkyl radical, comprising from 2 to 40 carbon atoms, preferably 6 to 14 carbon atoms, said radical optionally comprising one or more unsaturations,
- X is a linear or branched C₂-C₂₀ alkyl radical, having or not having unsaturations,
- Y represents an atom or a group selected from O, NH, S and C(O)O.

2. Polyurethane compound of formula (I) according to claim 1, in which at least one of the following conditions is fulfilled:
- A₁ represents a linear alkyl radical comprising from 2 to 12, preferably 2 to 6, in particular 3 carbon atoms, said radical optionally comprising one or more unsaturations;
- A₂ represents a linear or branched alkyl radical, comprising from 2 to 20 carbon atoms, in particular from 12 to 18 carbon atoms, said radical optionally comprising one or more unsaturations, and even more preferably a group of formula 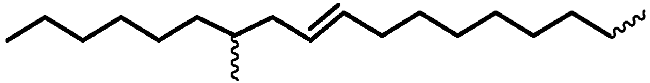
- I and m independently represent an integer from 1 to 10, the sum of I+m being, preferably, from 2 to 20;
- the sum of I+m has an average value of 5 to 12, in particular 7 or 11,
- n is an integer from 2 to 8;
- D is a linear or branched alkyl radical, comprising from 6 to 14 carbon atoms, said radical optionally comprising one or more unsaturations
- X is a linear or branched alkyl radical, having or not having unsaturations, comprising from 2 to 10, in particular 6 carbon atoms.
- Y represents an oxygen atom.

3. Polyurethane compound of formula (I) according to claim 1, in which
- A₁ represents a linear or branched alkyl radical comprising from 2 to 6, in particular 3 carbon atoms;
- A₂ represents a linear or branched alkyl radical, comprising from 12 to 18 carbon atoms, said radical optionally comprising one or more unsaturations;
- I and m represent, independently of one another, an integer from 1 to 10, the sum of I+m being, preferably, from 5 to 12;
- n is an integer from 2 to 8;
- D represents a linear or branched alkyl radical, comprising from 6 to 14 carbon atoms, said radical optionally comprising one or more unsaturations;
- X is a linear or branched alkyl radical, having or not having unsaturations, comprising from 2 to 10, in particular 6 carbon atoms, and
- Y represents an oxygen atom.

4. Polyurethane compound of formula (I) according to one of claims 1 to 3 in which A₁ represents a linear alkyl radical comprising 3 carbon atoms and/or A₂ represents a group of formula

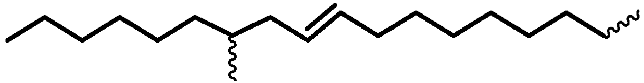

5. Polyurethane compound of formula (I) according to one of claims 1 to 4 having a content of natural origin greater than 80% according to standard NF-16128-2.

6. Process for preparing a polyurethane compound of formula (I) 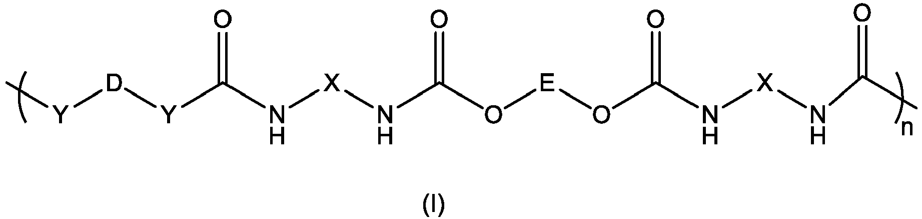 in which 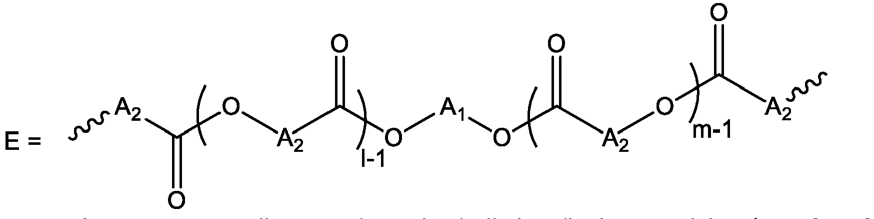
- A₁ represents a linear or branched alkyl radical, comprising from 2 to 20 carbon atoms, preferably from 2 to 12, and in particular from 2 to 6, said radical optionally comprising one or more unsaturations, optionally being interrupted by at least one heteroatom selected from O, N and S, and being optionally substituted by at least one substituant -OAlk, Alk representing an alkyl group comprising from 1 to 10 carbon atoms
- A₂ represents a linear or branched alkyl radical, comprising from 2 to 20 carbon atoms, preferably from 5 to 18, and preferentially from 6 to 17, said radical optionally comprising one or more unsaturations, and optionally being substituted by at least one substituent -OAlk, Alk representing an alkyl group comprising from 1 to 10 carbon atoms
- I and m represent, independently of one another, an integer from 1 to 20, preferably from 1 to 10, the sum of I+m being, preferably, from 2 to 20.
- n is an integer from 1 to 20, preferably 2 to 8,
- D represents a linear or branched alkyl radical, comprising from 2 to 40 carbon atoms, preferably 6 to 14 carbon atoms, said radical optionally comprising one or more unsaturations,
- X is a linear or branched C₂-C₂₀ alkyl radical, having or not having unsaturations,,
- Y represents an atom or a group selected from O, NH, S and C(O)O, comprising the following steps:
1) The functionalization of a di-OH estolide of formula (3) by a diisocyanate derivative of formula (4) in order to obtain a diisocyanate estolide of formula (5) 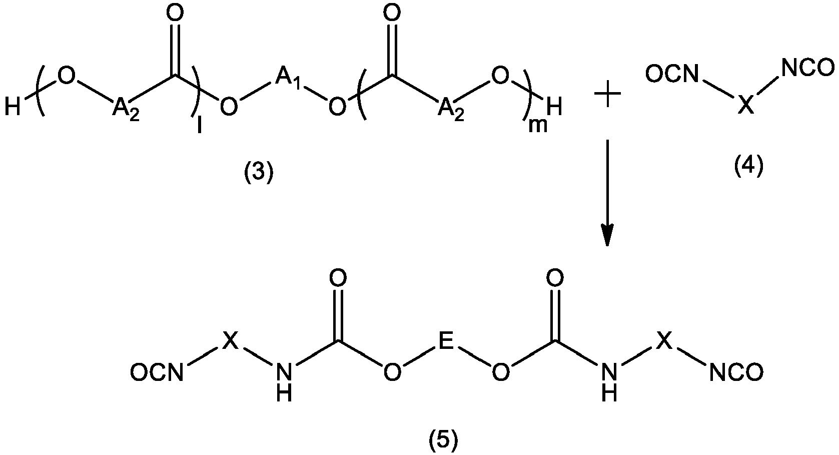 in which: 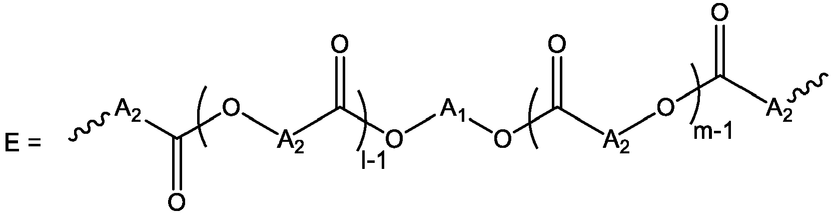
- A₁, A₂, I and m are as defined above;
- X is a linear or branched C₂-C₂₀ alkyl radical, having or not having unsaturations;
2) The chain extension in order to obtain a polyurethane of estolide of formula (7) by adding a diol of formula (6), optionally solubilized in an oil 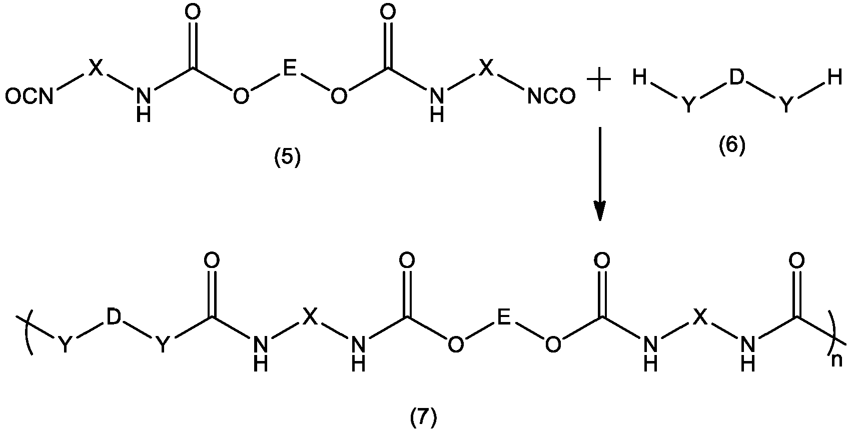 in which
- A₁, A₂, E, X, Y, I and m are as defined above;
- D represents a linear or branched alkyl radical, comprising from 2 to 40 carbon atoms, preferably from 6 to 14 carbon atoms, said radical optionally comprising one or more unsaturations;
- n is an integer from 1 to 20, preferably 2 to 8;
and
3) optionally, chain termination by addition of a nucleophilic molecule in excess capable of reacting with the residual isocyanate functions in order to give the gelling polyurethane of formula (I).

7. Process according to claim 6, in which at least one of the following conditions is fulfilled:
- A₁ represents a linear alkyl radical comprising from 2 to 12, preferably 2 to 6, in particular 3 carbon atoms, said radical optionally comprising one or more unsaturations;
- A₂ represents a linear or branched alkyl radical, comprising from 2 to 20 carbon atoms, in particular from 12 to 18 carbon atoms, said radical optionally comprising one or more unsaturations, and even more preferably a group of formula 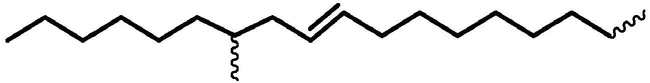
- I and m independently represent an integer from 1 to 10, the sum of I+m being, preferably, from 2 to 20;
- the sum of I+m has an average value of 5 to 12, in particular 7 or 11,
- n is an integer from 2 to 8;
- D is a linear or branched alkyl radical, comprising from 6 to 14 carbon atoms, said radical optionally comprising one or more unsaturations
- X is a linear or branched alkyl radical, having or not having unsaturations, comprising from 2 to 10, in particular 6 carbon atoms.
- Y represents an oxygen atom.

8. Process of one of claims 6 or 7 in which A₁ represents a linear alkyl radical comprising 3 carbon atoms, and/or in which A₂ represents a group of formula

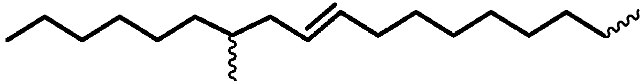

9. Process according to one of claims 6 to 8, in which the step of functionalization of the di-OH estolide by a diisocyanate derivative is carried out:
- by rapid addition of a quantity of diisocyanate (4) comprised between 1.4 and 2.0 equivalents with respect to 1 equivalent (eq) of di-OH estolide (3), calculated according to its OH index, at a temperature comprised between 60°C and 150°C, preferably between 110°C and 135°C, or
- by adding 1 equivalent (eq) of di-OH estolide (3) over a mixture of oil and diisocyanate (4) present in a quantity comprised between 1.4 and 2.0 equivalents, at a temperature greater than or equal to 110°C.

10. Process according to any one of claims 6 to 9, in which chain termination is carried out by the addition in excess of a nucleophilic molecule of the alcohol, thiol, amine or carboxylate type, mono- or difunctional, capable of reacting with the residual isocyanate functions in excess in the reaction medium at a temperature comprised between 110 and 150°C.

11. Use of the gelling polyurethane of formula (I) according to any one of claims 1 to 5, or obtained according to the process of any one of claims 6 to 10, as oil gelling agent.

12. Process for gelling at least one oil selected, for example, from the organic oils, including the vegetable oils, and the mixtures of apolar oils and more-polar oils, in which a gelling polyurethane of formula (I) according to any one of claims 1 to 5, or obtained according to the process of any one of claims 6 to 10, is added to said oil or oils, or to a composition containing it or them.

13. Gel containing or formed from the gelling polyurethane of formula (I) according to any one of claims 1 to 5, or from the gelling polyurethane of formula (I) obtained according to the process of any one of claims 6 to 10.

14. Gel according to claim 13, in which the content by weight of gelling polyurethane of formula (I) is comprised between 0.7% and 10%, preferably from 1 to 5%.

15. Pharmaceutical or cosmetic composition comprising a gel according to one of claims 13 or 14.
